# FASCICULE DE BREVET EUROPEEN

(11) **EP 3 860 719 B1**
(45) Date de publication et mention de la délivrance du brevet: **08.02.2023**
(21) Numéro de dépôt: 19795266.6
(22) Date de dépôt: 25.09.2019
(51) Int. Cl.: A61Q 5/12, A61K 8/06, C08F 220/60, C08F 220/56, A61K 8/81, C08F 2/32

(54) **COMPOSITION COSMÉTIQUE SOUS FORME D'ÉMULSION EAU-DANS-HUILE DESTINÉE AU TRAITEMENT DE CHEVEUX POUR AMÉLIORER LEUR PEIGNAGE**
KOSMETISCHE ZUSAMMENSETZUNG IN FORM EINER WASSER-IN-ÖL-EMULSION ZUR BEHANDLUNG DES HAARS ZUR VERBESSERUNG DER KÄMMBARKEIT
COSMETIC COMPOSITION IN THE FORM OF A WATER-IN-OIL EMULSION FOR TREATING HAIR TO IMPROVE COMBING

(30) Priorité: 01.10.2018 FR 1859055
(43) Date de publication de la demande: 11.08.2021
(73) Titulaire: Société d'Exploitation de Produits pour les Industries Chimiques SEPPIC, 75321 Paris cedex 07 (FR)
(72) Inventeur: CAVAILLES, Maïté, 81100 CASTRES (FR); BRETON, Marie-Laure, 81100 CASTRES (FR); MERAT, Emmanuelle, 81100 CASTRES (FR)
(74) Mandataire: Air Liquide
(86) Numéro de dépôt international: PCT/FR2019/052255
(87) Numéro de publication internationale: WO 2020/070410

(56) Documents cités:
- JP-A- 2001 002 925
- US-A1- 2002 146 438
- US-A1- 2004 162 405

## Description

La présente invention est relative à une composition cosmétique se présentant sous la forme d'une émulsion de type eau dans huile comprenant au moins un agent épaississant de type polyélectrolyte cationique réticulé, et destinée à être utilisée pour faciliter le peignage de mèches de cheveux.

Le cuir chevelu est protégé par un film hydrolipidique, situé à la surface de l'épiderme, et constitué de sébum et de sueur. Ce film hydrolipidique protège le cuir chevelu des agressions extérieures et de la déshydratation. Ce fil hydrolipidique qui agit comme un lubrifiant, se répartit le long de la tige pilaire et protège aussi les cheveux, en lissant les écailles fibres capillaires et en rendant alors le cheveu brillant et souple.

Lorsque les sécrétions de sébum sont insuffisantes, le film hydrolipidique se modifie, ce qui a pour conséquences une altération progressive de la cuticule et des cellules kératinisées qui perdent leur eau. On observe alors une forte diminution de l'élasticité de la kératine et un soulèvement des écailles des cheveux. La tige pilaire se dégrade jusqu'à l'intérieur de la fibre, et le cheveu devient sec et cassant.

Au niveau du cuir chevelu, suite aux sécrétions de sébum insuffisantes, la couche cornée se dessèche, perd de sa souplesse, ce qui provoque un inconfort cutané se manifestant par l'apparition d'irritations, de rougeurs et de tiraillements.

Sans ces sécrétions de sébum, le cuir chevelu devient lisse et parfois on observe la présence de squames (pellicules sèches).

Le follicule pileux des cheveux crépus produit peu de sébum. C'est pour cette raison que ces cheveux crépus sont souvent secs et déshydratés. Il est donc nécessaire de les « nourrir » plusieurs fois par semaine, avec des crèmes ou des huiles, en apportant ainsi des phases grasses sur les zones sèches du cuir chevelu.

Comparés aux cheveux caucasiens, les cheveux crépus se caractérisent par une déficience en eau accrue et le cuir chevelu, génétiquement ultra-sec, est plus vulnérable et très sensible. La sécrétion de sébum est physiologiquement faible pour tous les enfants jusqu'à la puberté, et pour les chevelures frisées et crépues, cette déficience de production de sébum se traduit par une lubrification insuffisante du cuir chevelu et la quasi-absence de film hydrolipidique et donc de protection de la fibre capillaire. La cuticule constituée de cellules plates qui se superposent comme des écailles, s'altère et la cohésion entre les cellules est rompue, il se produit une perte d'acides aminés dont le soufre. Au coiffage et au démêlage, le cuir chevelu devient douloureux, les cheveux forment facilement des noeuds et accrochent au peigne. Le temps consacré à les discipliner est plus long et constitue une gêne quotidienne pour le coiffage notamment des enfants.

L'objectif du traitement des cheveux après le lavage par l'intermédiaire de shampooings est de discipliner les cheveux, de diminuer la sécheresse du cuir chevelu, de diminuer la caractéristique cassante des cheveux, d'améliorer la brillance des cheveux, de faciliter le peignage et/ou le démêlage des cheveux.

Une solution, souvent employée par certaines ethnies africaines, consiste à utiliser des huiles seules. Ce mode de traitement possède des inconvénients majeurs liés au transfert du produit sur les doigts, sur les vêtements et oreiller ; de plus, les cheveux huilés accrochent davantage les poussières produisant un aspect inesthétique évident.

Une autre solution, notamment utilisée au Brésil, est d'utiliser des conditionneurs en émulsion ; notamment des émulsions de type huile-dans-Eau ou de type Eau-dans-Silicone qui contiennent des composés cationiques.

Ces conditionneurs sont faciles à appliquer et à utiliser, mais ils présentent l'inconvénient de ne pas procurer une efficacité suffisante.

Il existe donc un besoin de mettre au point des compositions pour le traitement des cheveux secs, du cuir chevelu, plus particulièrement pour des cheveux crépus, pour améliorer la facilité de coiffure des cheveux, pour diminuer la sécheresse du cuir chevelu, diminuer la caractéristique cassante des cheveux, améliorer la brillance des cheveux, et plus particulièrement pour faciliter le peignage et/ou le démêlage des cheveux.

Les solutions proposées dans l'art antérieur pour préparer des compositions cosmétiques pour le traitement des cheveux secs, du cuir chevelu, destinées à améliorer le peignage et/ou le démêlage de tels cheveux nécessitent d'impliquer des phases grasses spécifiques. Parmi ces phases grasses, on peut citer des phases grasses qui sont des dérivés siliconés, et qui peuvent être mises en oeuvre soit directement sur les cheveux, soit sous la forme d'un mélange huileux les contenant, soit sous la forme d'une émulsion les contenant.

Les émulsions sont des formes privilégiées car elles permettent de véhiculer en outre à la fois les substances hydrosolubles et des substances liposolubles qui sont couramment utilisées dans ces applications. On distingue les émulsions huile-dans-eau (H/E) dont la phase continue est constituée par une phase hydrophile, généralement une phase aqueuse, et la phase dispersée par une phase grasse lipophile, et les émulsions eau-dans-huile (E/H) dont la phase continue est constituée par une phase grasse lipophile et la phase dispersée par une phase hydrophile, généralement une phase aqueuse.

Le document US 2002/146 438 décrit trois compositions cosmétiques se présentant sous la forme d'une émulsion de type eau dans huile comprenant pour 100% de sa masse :
- de 60% à 95% massique d'une phase aqueuse gélifiée comprenant au moins un polyélectrolyte cationique réticulé : Plyquaternium-10,
de 5% à 40% massique d'une phase grasse comprenant au moins une huile et un système émulsionnant comprenant un agent tensioactif émulsionnant sélectionné parmi les polyhydroxystéarates de polyglycols : PEG-30 Dipolyhydroxystearate.Les émulsions huile-dans-eau sont intrinsèquement plus stables que les émulsions eau-dans-huile ; les émulsions eau-dans-huile présentent néanmoins de nombreux avantages. En effet, la séparation entre les gouttelettes d'eau réduit la possibilité de prolifération de micro-organismes. De plus, l'utilisation d'agents conservateurs, qui est essentielle lorsque la phase continue est aqueuse, peut être évitée, ou amoindrie, lorsque la phase continue est grasse. Les émulsions eau-dans-huile sont bien moins sensibles à une température basse que les émulsions huile-dans-eau. Enfin, pour des applications topiques à usage cosmétique, la demande de brevet Européen publiée sous le numéro EP 1961455 A1 divulgue qu'une phase continue huileuse permet de recouvrir la peau après application de l'émulsion eau-dans-huile, ce qui la protège de la déshydratation et contre des substances externes, en formant un film huileux persistant permettant ainsi de traiter les peaux sèches.

Les solutions proposées dans l'art antérieur pour préparer des émulsions cosmétiques destinées au traitement des cheveux et/ou du cuir chevelu se présentant sous la forme eau-dans-huile ne sont pas satisfaisantes car soit les dérivés siliconés employés sont volatils et peuvent présenter des effets néfastes envers l'environnement et les utilisateurs, soit les dérivés siliconés employés sont peu volatils et ils confèrent alors des propriétés sensorielles déplaisantes après application topique, comme par exemple des sensations de collant sur la peau.

Le problème technique est donc de développer une nouvelle composition se présentant sous la forme d'une émulsion de type eau dans huile qui ne se caractérise pas par les inconvénients exposés ci-dessus, qui reste homogène à température ambiante (25°C) et à 45°C après un stockage d'au minimum trois mois, et qui permet de faciliter le peignage et/ou le démêlage des cheveux.

Une solution de la présente invention est une composition cosmétique (C1) se présentant sous la forme d'une émulsion de type eau dans huile comprenant pour 100% de sa masse :
- de 60% à 95% massique d'une phase aqueuse gélifiée (A1) comprenant au moins un polyélectrolyte cationique réticulé qui comprend une proportion supérieure ou égale à 5% molaire d'unités monomériques issues d'un élément du groupe constitué par le chlorure de 2,N,N,N-tétraméthyl 2-[(1-oxo 2-propènyl) amino] propanammonium (AMPTAC), le chlorure de 2,N,N-triméthyl 2-[(1-oxo 2-propènyl) amino] propanammonium, le chlorure de N,N,N-triméthyl 3-[(1-oxo 2-propènyl) amino] propanammonium (APTAC).
- de 5% à 40% massique d'une phase grasse (A2) comprenant au moins une huile et un système émulsionnant comprenant une combinaison d'au moins un agent tensioactif émulsionnant (S) sélectionné parmi les éléments du groupe constitué par les compositions d'alkylpolyglycosides, les compositions d'alkylpolyglycosides et d'alcools gras, les esters de polyglycérols, les esters de polyglycérols alcoxylés, les polyhydroxystéarates de polyglycols, les polyhydroxystéarates de polyglycérols, les polyhydroxystéarates de polyglycérols alcoxylés.

Par polyélectrolyte cationique réticulé (PC), on désigne, dans la définition de la composition (C1) objet de la présente invention, un polyélectrolyte cationiquee réticulé non linéaire, se présentant à l'état de réseau tridimensionnel insoluble dans l'eau, mais gonflable à l'eau et conduisant à l'obtention d'un gel chimique.

Par huile, on désigne, dans la définition de la composition cosmétique (C1) objet de la présente invention, un composé et/ou un mélange de composés insoluble dans l'eau, et liquide à 25°C, et plus particulièrement :
- Les alcanes linéaires comportant de onze à dix-neuf atomes de carbone ;
- Les alcanes ramifiés, comportant de sept à quarante atomes de carbone, comme l'isododécane, l'isopentadécane, l'isohexadécane, l'isoheptadécane, l'isooctadécane, l'isononadécane ou l'isoeicosane), ou des mélanges de certains d'entre eux comme ceux cités ci-après et identifiés par leur nom INCI : C₇₋₈ isoparaffin, C₈₋₉ isoparaffin, C₉₋₁₁ isoparaffin, C₉₋₁₂ isoparaffin, C₉₋₁₃ isoparaffin, C₉₋₁₄ isoparaffin, C₉₋₁₆ isoparaffin, C₁₀₋₁₁ isoparaffin, C₁₀₋₁₂ isoparaffin, C₁₀₋₁₃ isoparaffin, C₁₁₋₁₂ isoparaffin, C₁₁₋₁₃ isoparaffin, C₁₁₋₁₄ isoparaffin, C₁₂₋₁₄ isoparaffin, C₁₂₋₂₀ isoparaffin, C₁₃₋₁₄ isoparaffin, C₁₃₋₁₆ isoparaffin ;
- Les cyclo-alcanes optionnellement substitués par un ou plusieurs radicaux alkyles linéaires ou ramifies ;
- Les huiles blanches minérales, comme celles commercialisées sous les noms suivants : Marcol^{™}52, Marcol^{™}82, Drakeol^{™}6VR, Eolane^{™}130, Eolane^{™}150 ;
- L'hémisqualane (ou 2,6,10-trimethyl- dodécane ; numéro CAS : 3891-98-3), le squalane (ou 2,6,10,15,19,23-hexamethyltetracosane), le polyisobutène hydrogéné ou le polydécène hydrogéné ;
- Les mélanges d'alcanes comportant de 15 à 19 atomes de carbone, lesdits alcanes étant des alcanes linéaires, des alcanes ramifiés et des cyclo-alcanes, et plus particulièrement le mélange (M₁) qui comprend pour 100% de sa masse, une proportion massique en alcanes ramifiés supérieure ou égale à 90% et inférieure ou égale à 100% ; une proportion massique en alcanes linéaires supérieure ou égale à 0% et inférieure ou égale à 9%, et plus particulièrement inférieure à 5% et une proportion massique en cyclo-alcanes supérieure ou égale à 0% et inférieure ou égale à 1%, par exemple les mélanges commercialisés sous les noms Emogreen^{™}L15 ou Emogreen^{™}L19 ;
- Les éthers d'alcool gras de formule (I) :

   Z₁-O-Z₂ (I),

   dans laquelle Z₁ et Z₂ identiques ou différents, représentent un radical alkyle linéaire ou ramifié comportant de cinq à dix-huit atomes de carbone, par exemple les dioctyl éther, didécyl éther, didodécyl éther, dodécyl octyl éther, dihexadécyl éther, (1,3-diméthyl butyl) tétradécyl éther, (1,3-diméthyl butyl) hexadécyl éther, le bis(1,3-diméthyl butyl) éther ou le dihexyl éther.
- Les mono-esters d'acides gras et d'alcools de formule (II) :

   R'₁-(C=O)-O-R'₂ (II),

   dans laquelle R'₁-(C=O) représente un radical acyle, saturé ou insaturé, linéaire ou ramifié, comportant de huit à vingt-quatre atomes de carbone, et R'2 représente, indépendamment de R'₁, une chaîne hydrocarbonée saturée ou insaturée, linéaire ou ramifiée comportant de un à vingt-quatre atomes de carbone, par exemple les laurate de méthyle, laurate d'éthyle, laurate de propyle, laurate d'isopropyle, laurate de butyle, laurate de 2-butyle, laurate d'hexyle, cocoate de méthyle, cocoate d'éthyle, cocoate de propyle, cocoate d'isopropyle, cocoate de butyle, cocoate de 2-butyle, cocoate d'hexyle, myristate de méthyle, myristate d'éthyle, myristate de propyle, le myristate d'isopropyle, le myristate de butyle, le myristate de 2-butyle, le myristate d'hexyle, le myristate d'octyle, le palmitate de méthyle, le palmitate d'éthyle, le palmitate de propyle, le palmitate d'isopropyle, le palmitate de butyle, le palmitate de 2-butyle, le palmitate d'hexyle, le palmitate d'octyle , l'oléate de méthyle, l'oléate d'éthyle, l'oléate de propyle, l'oléate d'isopropyle, l'oléate de butyle, l'oléate de 2-butyle, l'oléate d'hexyle, l'oléate d'octyle, le stéarate de méthyle, le stéarate d'éthyle, le stéarate de propyle, le stéarate d'isopropyle, le stéarate de butyle, le stéarate de 2-butyle, le stéarate d'hexyle, le stéarate d'octyle, l'isostéarate de méthyle, l'isostéarate d'éthyle, l'isostéarate de propyle, l'isostéarate d'isopropyle, l'isostéarate de butyle, l'isostéarate de 2-butyle, l'isostéarate d'hexyle, l'isostéarate d'isostéaryle ;
- Les di-esters d'acides gras et de glycérol de formule (III) et de formule (IV) :

   R'₃-(C=O)-O-CH₂-CH(OH)-CH₂-O-(C=O)-R'₄ (III)

   R'₅-(C=O)-O-CH₂-CH[O-(C=O)-R'₆]-CH₂-OH (IV),

   formules (III) (IV) dans lesquelles R'₃-(C=O), R'₄-(C=O), R's-(C=O), R'₆-(C=O), identiques ou différents, représentent un groupement acyle, saturé ou insaturé, linéaire ou ramifié, comportant de huit à vingt-quatre atomes de carbone ;
- Les tri-esters d'acides gras et de glycérol de formule (V) :

   R'₇-(C=O)-O-CH₂-CH[O-(C=O)-R"₈]-CH₂-O-(C=O)-R"₉ (V),

   dans laquelle R'₇-(C=O), R'₈-(C=O) et R'₉-(C=O), identiques ou différents, représentent un groupement acyle, saturé ou insaturé, linéaire ou ramifié, comportant de huit à vingt-quatre atomes de carbone.
- Les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula, les huiles issues de fleurs ou de légumes ;
- Les huiles végétales éthoxylées.

En plus de l'huile, la composition cosmétique selon l'invention peut comprendre une cire.

Lorsque la composition cosmétique (C1) selon l'invention comprend une cire, cette dernière est plus particulièrement choisie parmi la cire d'abeille, la cire de carnauba, la cire de candelilla, la cire d'ouricoury, la cire du Japon, la cire de fibre de liège, la cire de canne à sucre, les cires de paraffines, les cires de lignite, les cires microcristallines, la cire de lanoline; l'ozokérite; la cire de polyéthylène; les cires de silicone ; les cires végétales ; les alcools gras et les acides gras solides à température ambiante; les glycérides solides à température ambiante.

De préférence, la composition selon l'invention comprend au moins une huile choisie parmi les éléments du groupe constitué par l'huile de ricin, les huiles de paraffine, l'huile d'olive, le cocoyl caprylate/caprate, le myristate d'isopropyle, le capric caprylic triglycéride.

Selon le cas, la composition selon l'invention peut comprendre une ou plusieurs des caractéristiques ci-dessous :
- la phase aqueuse gélifiée (A1) comprend pour 100% de sa masse de 0,1% à 7% massique d'un polyélectrolyte cationique réticulé (PC) et de 93% à 99,9% massique d'eau, de préférence de 0,5% à 5% massique d'un polyélectrolyte cationique réticulé (PC) et de 95% à 99,5% massique d'eau, encore plus préférentiellement de 0,5% à 3% massique d'un polyélectrolyte cationique réticulé (PC) et de 97% à 99,5% massique d'eau
- le polyélectrolyte cationique réticulé (PC) comprend pour 100% molaire :
   a1) de 5% molaire à 60% molaire, de préférence de 40% à 60% molaire, et encore plus préférentiellement de 45% molaire à 60% molaire d'unités monomériques issues d'un élément du groupe constitué par le chlorure de 2,N,N,N-tétraméthyl 2-[(1-oxo 2-propènyl) amino] propanammonium (AMPTAC), le chlorure de 2,N,N-triméthyl 2-[(1-oxo 2-propènyl) amino] propanammonium, le chlorure de N,N,N-triméthyl 3-[(1-oxo 2-propènyl) amino] propanammonium (APTAC),
   a2) de 0,1 % molaire à 5 % molaire, de préférence de 0,1% à 3% molaire, et encore plus préférentiellement de 0,5% molaire à 3% molaire d'unités monomériques issues d'au moins un monomère de formule (VI) : 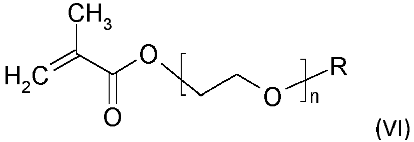 dans laquelle R représente un radical alkyle linéaire ou ramifié comportant de huit à vingt atomes de carbone et n représente un nombre entier supérieur ou égal à zéro et inférieur ou égal à vingt ;
   a3) de 35 % molaire à 94,9 % molaire, de préférence de 37% à 59,9% molaire, et encore plus préférentiellement de 37% molaire à 54,5% molaire d'unités monomériques issues d'au moins un monomère neutre choisi parmi les éléments du groupe constitué par l'acrylamide, le N,N-diméthyl acrylamide, le méthacrylamide, le N-isopropyl acrylamide, le 2-hydroxyétyl acrylate ou le 2-hydroxyéthyl méthacrylate ;
   a4) d'une proportion supérieure à 0% molaire et inférieure ou égale à 1% molaire d'unités monomériques issues d'au moins un monomère de réticulation (AR) diéthylénique ou polyéthylénique ; la somme des dites proportions molaires en unités monomériques selon a1), a2), a3) et a4) étant égale à 100% molaire.
- le polyélectrolyte cationique réticulé (PC) comprend pour 100% molaire de 5% molaire à 60% molaire, plus particulièrement de 40% à 60% molaire, et encore plus particulièrement de 45% molaire à 60% molaire d'unités monomériques issues du chlorure de N,N,N-triméthyl 3-[(1-oxo 2-propènyl) amino] propanammonium (APTAC).
- dans la composition (C1) telle que définie ci-dessus, ledit monomère neutre est choisi parmi l'acrylamide, le N,N-diméthyl acrylamide, le 2-hydroxyétyl acrylate ou le 2-hydroxyéthyl méthacrylate ; le monomère neutre sera de préférence le 2-hydroxyétyl acrylate.
- dans la formule (VI) du monomère présent dans ledit polyélectrolyte cationique réticulé (PC) compris dans la composition (C1) objet de la présente invention, par radical alkyle linéaire ou ramifié comportant de huit à vingt atomes de carbone, on désigne plus particulièrement pour R :
   - ou bien un radical dérivé des alcools primaires linéaires tels que par exemple, le radical octyle, décyle, undécyle, dodécyle, tridécyle, tétradécyle, pentadécyle, hexadécyle, heptadécyle, octadécyle, nonadécyle ou eicosyle ;
   - ou bien un radical dérivé des alcools de Guerbet, qui sont des 1-alcanols ramifiés répondant à la formule générale :

      CH₃-(CH₂)ₚ-CH[CH₃-(CH₂)ₚ₋₂]-CH₂OH,

      dans laquelle p représente un nombre entier compris entre 2 et 9, tels que, par exemple, les radicaux 2-éthyl hexyle, 2-propyl heptyle, 2-butyl octyle, 2-pentyl nonyle, 2-hexyl décyle ou 2-octyl dodécyle ;
   - ou bien un radical dérivé des isoalcanols répondant à la formule générale :

      CH₃-CH(CH₃)-(CH₂)ₘ-CH₂OH,

      dans laquelle m représente un nombre entier compris entre 2 et 16, tels que, par exemple, les radicaux 4-méthyl pentyle, 5-méthyl hexyle, 6-méthyl heptyle, 15-méthyl pendadécyle ou 16-méthyl heptadécyle, soit les radicaux 2-hexyl octyle, 2-octyl décyle ou 2-hexyl dodécyle.
- dans la formule (VI), R représente un radical alkyle comportant de 12 à 18 atomes de carbone ;
- dans la formule (VI), R représente un radical alkyle choisi parmi les éléments du groupe constitué par le radical dodécyle, tridécyle, tétradécyle, hexadécyle, octadécyle ;
- dans la formule (VI), n représente un nombre entier supérieur ou égal à 3 et inférieur ou égal à 20 ;
- ledit monomère de formule (VI) est le méthacrylate de lauryle tétraéthoxylé ;
- ledit monomère de formule (VI) est le méthacrylate de stéaryle eicosaéthoxylé ;
- ledit monomère de réticulation (AR) est mis en oeuvre dans une proportion molaire inférieure ou égale à 0,5%, plus particulièrement inférieure ou égale à 0,25% et tout particulièrement inférieure ou égales à 0,1% ; elle est plus particulièrement supérieure ou égales à 0,005% molaire ;
- ledit polyélectrolyte cationique réticulé (PC) est choisi parmi les terpolymères du chlorure de N,N,N-triméthyl 3-[(1-oxo 2-propènyl) amino] propanammonium (APTAC), du 2-hydroxyéthyl acrylate et du méthacrylate de lauryle tétraéthoxylé, réticulé au triméthylol propanetriacrylate ou les terpolymères du chlorure de N,N,N-triméthyl 3-[(1-oxo 2-propènyl) amino] propanammonium (APTAC), du 2-hydroxyéthyl acrylate et du méthacrylate de stéaryle eicosaéthoxylé, réticulé au triméthylol propanetriacrylate ;
- le polyélectrolyte cationique réticulé (PC) est un terpolymère de chlorure de N,N,N-triméthyl 3-[(1-oxo 2-propènyl) amino] propanammonium (APTAC), du 2-hydroxyéthyl acrylate et du méthacrylate de lauryle tétraéthoxylé, réticulé au triméthylol propanetriacrylate ou au diméthacrylate d'éthylèneglycol ou au diacrylate d'éthylèneglycol.
- ledit polyélectrolyte cationique réticulé (PC) comporte pour 100% molaire :
   - De 45% molaire à 60% molaire d'unités monomériques issues du chlorure de N,N,N-triméthyl 3-[(1-oxo 2-propènyl) amino] propanammonium
   - De 0,5% molaire à 3% molaire d'unités monomériques issues du méthacrylate de lauryle tétraéthoxylé, et
   - De 37% molaire à 54,5% molaire d'unités monomériques issues du 2-hydroxyéthyl acrylate.
- ledit système émulsionnant (S) consiste en une composition (C2) d'alkylpolyglycosides représentée par la formule (VII) :

   R₁-O-(G)ₓ-H (VII)

   dans laquelle x représente un nombre décimal compris entre 1,05 et 2,5, G représente le reste du xylose, et R₁ représente le radical 2-octyl dodécyle, ladite composition (C₁) consistant en un mélange de composés représentés par les formules (VII₁), (VII₂), (VII₃), (VII₄) et (VII₅) :

   R₁-O-(G)₁-H (VII₁)

   R₁-O-(G)₂-H (VII₂)

   R₁-O-(G)₃-H (VII₃)

   R₁-O-(G)₄-H (VII₄)

   R₁-O-(G)₅-H (VII₅)

   dans les proportions molaires respectives a₁, a₂, a₃, a₄ et a₅, telles que :
   - La somme a₁+ a₂ + a₃ + a₄ + a₅ est égale à 1 et que
   - La somme a₁ + 2a₂ + 3a₃ + 4a₄ + 5a₅ est égale à x.
- ledit système émulsionnant (S) consiste en une composition (C3) comprenant pour 100% de leur masse :
   - De 10% à 50% massique d'au moins une composition d'alkylpolyglycosides (C2) représentée par la formule (VII) :

      R₁-O-(G)ₓ-H (VII)

      dans laquelle x représente un nombre décimal compris entre 1,05 et 2,5, G représente le reste du xylose et R₁ représente le radical 2-octyl dodécyle, ladite composition consistant en un mélange de composés représentés par les formules (VII₁), (VII₂), (VII₃), (VII₄) et (VII₅) :

      R₁-O-(G)₁-H (VII₁)

      R₁-O-(G)₂-H (VII₂)

      R₁-O-(G)₃-H (VII₃)

      R₁-O-(G)₄-H (VII₄)

      R₁-O-(G)₅-H (VII₅)

      dans les proportions molaires respectives a₁, a₂, a₃, a₄ et a₅, telles que :
      - La somme a₁+ a₂ + a₃ + a₄ + a₅ est égale à 1 et que
      - La somme a₁ + 2a₂ + 3a₃ + 4a₄ + 5a₅ est égale à x ; et
   - De 90% à 50% massique d'au moins un alcool gras de formule (VIII) :

      R'₁-OH (VIII),

      dans laquelle R'₁ représente le radical 2-octyl dodécyle.
- ledit système émulsionnant (S) consiste en une composition (C4) comprenant pour 100% de sa masse :
   - de 15% à 25% massique d'au moins une composition (C2) représentée par la formule (VII) :

      R1-O-(G)ₓ-H (VII)

      dans laquelle x représente un nombre décimal compris entre 1,05 et 2,5, G représente le reste du xylose et R1 représente le radical 2-octyl dodécyle, ladite composition (C₁) consistant en un mélange de composés représentés par les formules (VII₁), (VII₂), (VII₃), (VII₄) et (VII₅) :

      R₁-O-(G)₁-H (VII₁)

      R₁-O-(G)₂-H (VII₂)

      R₁-O-(G)₃-H (VII₃)

      R₁-O-(G)₄-H (VII₄)

      R₁-O-(G)₅-H (VII₅)

      dans les proportions molaires respectives a₁, a₂, a₃, a₄ et a₅, telles que :
      - La somme a₁+ a₂ + a₃ + a₄ + a₅ est égale à 1 et que
      - La somme a₁ + 2a₂ + 3a₃ + 4a₄ + 5a₅ est égale à x ;
   - de 55% à 65% massique d'au moins un alcool gras de formule (VIII) :

      R'₁-OH (VIII),

      dans laquelle R'₁ représente le radical 2-octyl dodécyle ;
   - de 10% à 30% massique d'au moins un polyhydroxystéarates de polyglycols représenté par la formule (XI) : 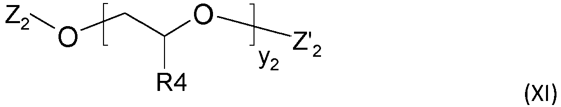
   dans laquelle y₂ représente un nombre entier supérieur ou égal à 2 et inférieur ou égal à 50, R₄ représente l'atome d'hydrogène, le radical méthyle ou le radical éthyle, Z₂ représente un radical de formule (XII) : 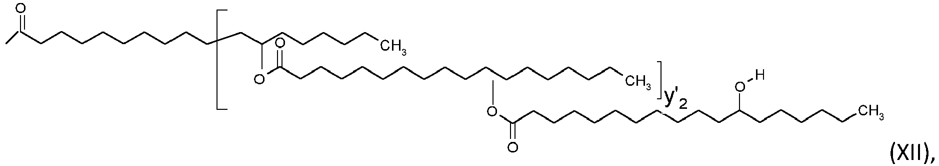 dans laquelle y'₂ représente un nombre entier supérieur ou égal à 0 et inférieur ou égal à 10, plus particulièrement supérieur ou égal à 1 et inférieur ou égal à 10, Z'₂ représente un radical de formule (XII) telle que définie ci-dessus, avec Z₂' identique ou différent de Z₂, ou l'atome d'hydrogène.

Par au moins un monomère de réticulation (AR) diéthylènique ou polyéthylénique, on désigne, dans la définition dudit polyélectrolyte cationique réticulé (PC), on désigne notamment un monomère choisi parmi les éléments du groupe constitué par le méthylène-bis(acrylamide), le diméthacrylate d'éthylèneglycol, le diacrylate de diéthylèneglycol, le diacrylate d'éthylèneglycol, le diallyl urée, le triallylamine, le triméthylol propanetriacrylate, l'acide diallyoxyacétique ou un de ses sels comme le diallyloxyacétate de sodium, ou un mélange de ces composés ; et plus particulièrement un monomère choisi parmi le diméthacrylate d'éthylèneglycol, le triallylamine, le triméthylol propanetriacrylate ou le méthylène-bis(acrylamide) ou un mélange ces composés.

Le polyélectrolyte cationique réticulé (PC) mis en oeuvre dans la composition cosmétique (C1) objet de la présente invention peut être préparé par la mise en oeuvre d'un procédé de polymérisation radicalaire connu de l'homme du métier, comme par exemple les procédés de polymérisation en solution, de polymérisation en suspension, de polymérisation en suspension inverse, de polymérisation en émulsion, de polymérisation en émulsion inverse, de polymérisation en milieu solvant suivie d'une étape de précipitation du polymère formé.

Selon un aspect plus particulier, le polyélectrolyte cationique réticulé (PC) mis en oeuvre dans la composition cosmétique (C1) objet de la présente invention peut être préparé par la mise en oeuvre d'un procédé de polymérisation en milieu solvant suivie d'une étape de précipitation du polymère formé, ou de polymérisation en émulsion inverse optionnellement suivi d'une étape de concentration et/ou d'atomisation.

Selon un aspect plus particulier, le polyélectrolyte cationique réticulé (PC) mis en oeuvre dans la composition cosmétique (C1) objet de la présente invention peut être préparé selon un des procédés décrits ci-dessus et impliquer l'utilisation d'agents de transfert ou limiteurs de chaîne. Les agents de transfert ou limiteurs de chaîne sont plus particulièrement choisi parmi le groupe constitué par l'hypophosphite de sodium, des alcools de faibles poids moléculaires par exemple le méthanol, l'éthanol, le propanol-1, l'isopropanol, le butanol, des thiols, par exemple le 2-mercapto éthanol, des agents de transfert comprenant une fonction sulfate, par exemple le methallylsulfonate de sodium, ou des mélanges desdits agents de transfert. Les agents de transfert ou limiteurs de chaînes sont plus particulièrement utilisés dans des proportions molaires, exprimées par rapport au nombre total de moles de monomères mis en oeuvre, de 0,001% à 1% molaire, plus particulièrement de 0,001% à 0,5% molaire, et tout particulièrement de 0,001% à 0,1% molaire.

Dans la définition de la composition cosmétique (C1) objet de la présente invention, on désigne par compositions d'alkyl polyglycosides, une composition (C2) représentée par la formule (VII) :

R₁-O-(G)ₓ-H (VII)

dans laquelle x représente un nombre décimal compris entre 1,05 et 5, G représente le reste d'un sucre réducteur, et R₁ représente un radical aliphatique hydrocarboné, saturé ou insaturé, linéaire ou ramifié, éventuellement substitué avec un ou plusieurs groupe hydroxyle, comportant de 12 à 36 atomes de carbone, ladite composition (C2) consistant en un mélange de composés représentés par les formules (VII₁), (VII₂), (VII₃), (VII₄) et (VII₅) :

R₁-O-(G)₁-H (VII₁)

R₁-O-(G)₂-H (VII₂)

R₁-O-(G)₃-H (VII₃)

R₁-O-(G)₄-H (VII₄)

R₁-O-(G)₅-H (VII₅)

dans les proportions molaires respectives a₁, a₂, a₃, a₄ et a₅, telles que :
- La somme a₁+ a₂ + a₃ + a₄ + a₅ est égale à 1 et que
- La somme a₁ + 2a₂ + 3a₃ + 4a₄ + 5a₅ est égale à x.

Par radical aliphatique hydrocarboné, saturé ou insaturé, linéaire ou ramifié, comportant de 12 à 36 atomes de carbone, éventuellement substitué avec un ou plusieurs groupes hydroxyle, on désigne pour le radical R₁ dans la formule (VII) telle que définie ci-dessus :
- Les radicaux alkyle linéaires saturés, par exemple les radicaux n-dodécyle, n-tridécyle, n-tétradécyle, n-pentadécyle, n-hexadécyle, n-heptadécyle, n-octadécyle, n-nonadécyle, n-eicosyle, n-docosyle ;
- Les radicaux linéaires insaturés tels que les radicaux dodécènyle, tridécènyle, tétradécènyle, pentadécènyle, hexadécènyle, heptadécènyle, octadécènyle, nonadécènyle, eicosènyle, docosènyle, 4-dodécènyle, ou 5-dodécènyle ;
- Les radicaux aliphatiques saturés ou insaturés, linéaires ou ramifiés, comportant de 12 à 36 atomes de carbone substitués par un ou deux groupes hydroxy, tels que les radicaux hydroxydodécyle, hydroxytétradécyle, hydroxyhexadécyle, hydroxyoctadécyle, hydroxyeicosyle, hydroxydocosyle, par exemple le radical 12-hydroxy octadécyle.
- Les radicaux issus des isoalcanols de formule (1) :

   (CH₃)(CH₃)CH-(CH₂)ᵣ-CH₂-OH (1)

   dans laquelle r représente un nombre entier compris entre 8 et 20, par exemple les radicaux isodécyle, isoundécyle, isododécyle, isotridécyle, isotétradécyle, isopentadécyle, isohexadécyle, isopentadécyle, isooctadécyle, isononadécyle, isoeicosyle ou isodocosyle ; Les radicaux alkyles ramifiés, issus des alcools de Guerbet, de formule (2) :

   CH(CₛH₂ₛ₊₁)(CₜH₂ₜ₊₁)-CH₂-OH (2)

   dans laquelle t est un nombre entier compris entre 6 et 18, s est un nombre entier compris entre 4 et 18 et la somme s + t est supérieure ou égale à 10, et inférieure ou égale à 22, par exemple les radicaux 2-butyl octyle, 2-butyl décyle, 2-hexyl octyle, 2-hexyl décyle, 2-octyl décyle, 2-hexyl dodécyle, 2-octyl dodécyle, 2-décyl tétradécyle, 2-dodécyl hexadécyle, 2-tétradécyl octadécyle.

Selon un aspect particulier, dans la définition de la formule (VII) telle que définie ci-dessus, R₁ représente un radical aliphatique hydrocarboné, saturé ou insaturé, linéaire ou ramifié comportant de 12 à 24 atomes de carbone.

Par sucre réducteur, dans la définition de la formule (VII) telle que définie ci-dessus, on désigne les dérivés saccharidiques qui ne présentent pas dans leurs structures de liaison glycosidique établie entre un carbone anomérique et l'oxygène d'un groupement acétal tels qu'ils sont définis dans l'ouvrage de référence : « Biochemistry », Daniel Voet/Judith G. Voet, p. 250, John Wyley & Sons, 1990. La structure oligomérique (G)ₓ, peut se présenter sous toutes formes d'isoméries, qu'il s'agisse d'isomérie optique, d'isomérie géométrique ou d'isomérie de position ; elle peut aussi représenter un mélange d'isomères.

Dans la formule (VII) telle que définie ci-dessus, le groupe R₁-O- est lié à G par le carbone anomérique du reste saccharide, de manière à former une fonction acétal.

Selon un aspect particulier dans la définition de la formule (VII) telle que définie ci-dessus, G représente le reste d'un sucre réducteur choisi parmi le glucose, le dextrose, le saccharose, le fructose, l'idose, le gulose, le galactose, le maltose, l'isomaltose, le maltotriose, le lactose, le cellobiose, le mannose, le ribose, le xylose, l'arabinose, le lyxose, l'allose, l'altrose, le dextrane ou le tallose; et plus particulièrement G représente le reste d'un sucre réducteur choisi parmi les restes du glucose, du xylose et de l'arabinose.

Selon un aspect encore plus particulier, dans la définition de la formule (VII) représentant la composition (C2) comprise dans la composition cosmétique (C1) objet de la présente invention, x représente un nombre décimal supérieur ou égal à 1,05 et inférieur ou égal à 2,5, plus particulièrement supérieur ou égal à 1,05 et inférieur ou égal à 2,0, et encore plus particulièrement supérieur ou égal à 1,25 et inférieur ou égal à 2,0.

Selon un aspect encore plus particulier, dans la définition de la formule (VII) telle que définie ci-dessus, R₁ représente le radical choisi parmi au moins un des éléments du groupe constitué par les radicaux n-dodécyle, n-tétradécyle, n-hexadécyle, n-octadécyle, n-eicosyle, n-docosyle, 2-hexyl décyle, 2-octyl décyle, 2-hexyl dodécyle, 2-octyl dodécyle, 2-décyl tétradécyle ; G représente le reste d'un sucre réducteur choisi parmi les restes du glucose et du xylose et x représente un nombre décimal supérieur ou égal à 1,05 et inférieur ou égal à 2,5.

Dans la définition de la composition cosmétique (C1) objet de la présente invention, on désigne par composition d'alkylpolyglycosides et d'alcools gras, une composition (C3) comprenant pour 100% de sa masse :
- De 10% à 50% massique, plus particulièrement de 15% à 40% massique, et encore plus particulièrement de 20% à 30% massique, d'au moins une composition (C2) représentée par la formule (II) telle que définie précédemment,
- De 90% à 50% massique, plus particulièrement de 85% à 60% massique, et encore plus particulièrement de 80% à 70% massique, d'au moins un alcool gras de formule (VIII) :

   R'₁-OH (VIII),

   dans laquelle R'₁, identique ou différent de R₁, représente un radical aliphatique hydrocarboné, saturé ou insaturé, linéaire ou ramifié, éventuellement substitué avec un ou plusieurs groupe hydroxyle, comportant de 12 à 36 atomes de carbone.

Selon un aspect particulier, dans la définition de la formule (VII) représentant la composition (C2) comprise dans la composition (C3), R₁ représente le radical choisi parmi les radicaux n-dodécyle, n-tétradécyle, n-hexadécyle, n-octadécyle, n-eicosyle, n-docosyle, 2-hexyl décyle, 2-octyl décyle, 2-hexyl dodécyle, 2-octyl dodécyle, 2-décyl tétradécyle, G représente le reste d'un sucre réducteur choisi parmi les restes du glucose et du xylose et x représente un nombre décimal supérieur ou égal à 1,05 et inférieur ou égal à 2,5.

Selon un aspect plus particulier, dans la définition de la formule (II) représentant la composition (C2) comprise dans la composition (C3), R₁ représente le radical 2-octyl dodécyle, G représente le reste du xylose et x représente un nombre décimal supérieur ou égal à 1,05 et inférieur ou égal à 2,5.

Selon un aspect plus particulier, dans la définition de l'alcool gras de formule (VIII) telle que définie ci-dessus, R'₁ représente un radical choisi parmi les radicaux n-dodécyle, n-tétradécyle, n-hexadécyle, n-octadécyle, n-eicosyle, n-docosyle, 2-hexyl décyle, 2-octyl décyle, 2-hexyl dodécyle, 2-octyl dodécyle ou 2-décyl tétradécyle, R'₁ représente tout particulièrement le radical 2-octyl dodécyle.

Dans la définition de la composition cosmétique (C1) objet de la présente invention, on désigne par ester de polyglycérol, un composé de formule (IX) : 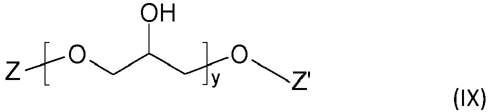 dans laquelle Z représente un radical acyle de formule R₂-C(=O)-, dans laquelle R₂ représente un radical aliphatique hydrocarboné, saturé ou insaturé, linéaire ou ramifié, comportant de 11 à 35 atomes de carbone et plus particulièrement un radical choisi parmi les radicaux dodécanoyle, tétradécanoyle, hexadécanoyle, octadécanoyle, eicosanoyle, docosanoyle, oléyle, linoléyle, linolénoyle ou isostéaryle, Z' représente le radical acyle de formule R₂-C(=O)- tel que défini ci-dessus, avec Z' identique ou différent de Z, ou l'atome d'hydrogène, et y représente un nombre entier supérieur ou égal à 2 et inférieur ou égal à 20.

Selon un aspect plus particulier, le composé de formule (IX) est choisi parmi les éléments du groupe constitué par l'oléate de décaglycérol, l'isostéarate de décaglycérol, le monolaurate de décaglycérol, le mono-linoléate de décaglycérol, le mono-myristate de décaglycérol.

Dans la définition de la composition cosmétique (C1) objet de la présente invention, on désigne par esters de polyglycérols alcoxylés, un composé de formule (X) : 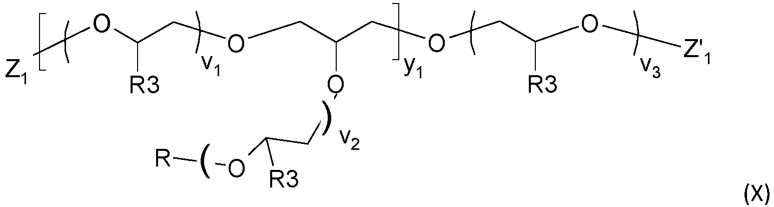 dans laquelle Z₁ représente un radical acyle de formule R'₂-C(=O)-, dans laquelle R'₂ représente un radical aliphatique hydrocarboné, saturé ou insaturé, linéaire ou ramifié, comportant de 11 à 35 atomes de carbone, et plus particulièrement un radical choisi parmi les radicaux radicaux dodécanoyle, tétradécanoyle, hexadécanoyle, octadécanoyle, eicosanoyle, docosanoyle, oléyle, linoléyle, linolénoyle ou isostéaryle, Z₁' représente le radical acyle de formule R'₂-C(=O)- tel que défini ci-dessus, avec Z₁' identique ou différent de Z₁, ou l'atome d'hydrogène, R₃ représente l'atome d'hydrogène, le radical méthyle, ou le radical éthyle, y₁ représente un nombre entier supérieur ou égal à 2 et inférieur ou égal à 20, v₁, v₂, v₃, identiques ou différents, représentent un nombre entier supérieur ou égal à 0 et inférieur ou égal à 50, et la somme [(y₁. v₁) + (y₁. v₂) + v₃)] est un nombre entier supérieur ou égal à 1 et inférieur ou égal à 50.

Dans la définition de la composition cosmétique (C1) objet de la présente invention, on désigne par polyhydroxystéarates de polyglycols, un composé de formule (XI) : 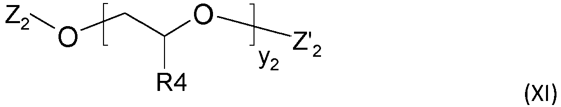 dans laquelle y₂ représente un nombre entier supérieur ou égal à 2 et inférieur ou égal à 50, R₄ représente l'atome d'hydrogène, le radical méthyle, ou le radical éthyle, Z₂ représente un radical de formule (XII) : 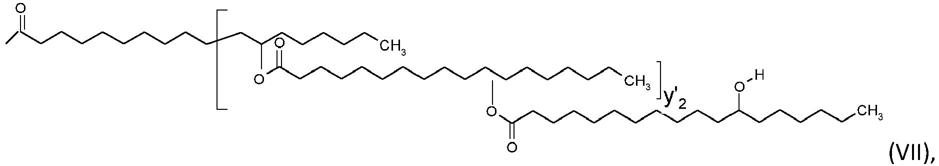 dans laquelle y'₂ représente un nombre entier supérieur ou égal à 0 et inférieur ou égal à 10, plus particulièrement supérieur ou égal à 1 et inférieur ou égal à 10 et Z'₂ représente un radical de formule (XII) telle que définie ci-dessus, avec Z₂' identique ou différent de Z₂, ou l'atome d'hydrogène.

Dans la définition de la composition cosmétique (C1) objet de la présente invention, on désigne par polyhydroxystéarate de polyglycérol, un composé représenté par la formule (XIII) : 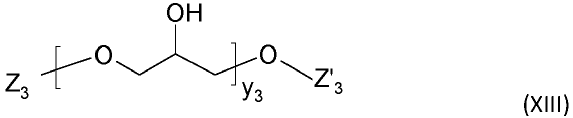 dans laquelle Z₃ représente un radical de formule (XII) telle que définie ci-dessus, Z'₃ représente un radical de formule (XII) telle que définie ci-dessus, avec Z₃' identique ou différent de Z₃, ou l'atome d'hydrogène, y₃ représente un nombre entier supérieur ou égal à 2 et inférieur ou égal à 20.

Dans la définition de la composition cosmétique (C1) objet de la présente invention, on désigne par polyhydroxystéarate de polyglycérol alcoxylé, un composé représenté par la formule (XIV) : 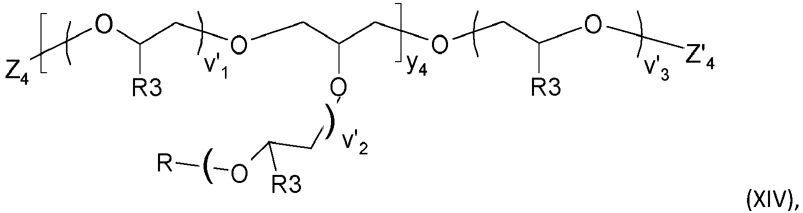 dans laquelle Z₄ représente un radical de formule (XII) telle que définie ci-dessus, Z'₄ représente un radical de formule (XII) telle que définie ci-dessus, avec Z₄' identique ou différent de Z₄, ou l'atome d'hydrogène, y₄ représente un nombre entier supérieur ou égal à 2 et inférieur ou égal à 20, v'₁, v'₂, v'₃, identiques ou différents, représentent un nombre entier supérieur ou égal à 0 et inférieur ou égal à 50, et la somme [(y₄. v'₁) + (y₄. v'₂) + v'₃)] est un nombre entier supérieur ou égal à 1 et inférieur ou égal à 50.

Dans la définition de la composition cosmétique (C1) objet de la présente invention, on désigne par copolymère polyéthylèneglycol-alkylglycol, un composé représenté par la formule (XV) : 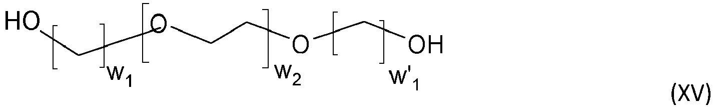 dans laquelle w₁ et w'₁, identiques ou différents, représentent un nombre entier supérieur ou égal à 1 et inférieur ou égal à 50, plus particulièrement supérieur ou égal à 1 et inférieur ou égal à 25, w₂ représente un nombre entier supérieur ou égal à 1 et inférieur ou égal à 100, plus particulièrement supérieur ou égal à 1 et inférieur ou égal à 50.

La présente invention a également pour objet un polyélectrolyte cationique réticulé (PC1) comprenant pour 100% molaire :
b1) de 40% à 60% molaire, et encore plus particulièrement de 45% molaire à 60% molaire d'unités monomériques cationiques issues d'un élément du groupe constitué par le chlorure de 2,N,N,N-tétraméthyl 2-[(1-oxo 2-propènyl) amino] propanammonium (AMPTAC), le chlorure de 2,N,N-triméthyl 2-[(1-oxo 2-propènyl) amino] propanammonium, le chlorure de N,N,N-triméthyl 3-[(1-oxo 2-propènyl) amino] propanammonium (APTAC),
b2) de 0,1% à 3% molaire, et encore plus particulièrement de 0,5% molaire à 3% molaire d'unités monomériques issues d'au moins un monomère de formule (VI) : 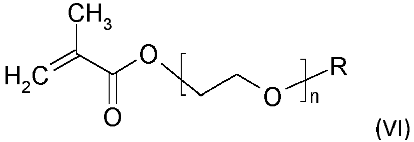 dans laquelle R représente un radical alkyle linéaire ou ramifié comportant de huit à vingt atomes de carbone et n représente un nombre entier supérieur ou égal à zéro et inférieur ou égal à vingt ;
b3) de 37% à 59,9% molaire, et encore plus particulièrement de 37% molaire à 54,5% molaire d'unités monomériques issues d'au moins un monomère neutre choisi parmi les éléments du groupe constitué par l'acrylamide, le N,N-diméthyl acrylamide ; le méthacrylamide ou le N-isopropyl acrylamide le 2-hydroxyétyl acrylate ou le 2-hydroxyéthyl méthacrylate ;
b4) d'une proportion supérieure à 0% molaire et inférieure ou égale à 1% molaire d'unités monomériques issues d'au moins un monomère de réticulation (AR) diéthylénique ou polyéthylénique ;
la somme des dites proportions molaires en unités monomériques selon b1), b2), b3) et b4) étant égale à 100% molaire.

Selon le cas, le polyélectrolyte cationique réticulé (PC1) selon l'invention peut présenter une ou plusieurs des caractéristiques suivantes :
- le polyélectrolyte cationique réticulé (PC1) est un polyélectrolyte cationique réticulé (PC1) pour lequel les unités monomériques cationiques sont issues du chlorure de N,N,N-triméthyl 3-[(1-oxo 2-propènyl) amino] propanammonium (APTAC),
- le polyélectrolyte cationique réticulé (PC1) est un polyélectrolyte cationique réticulé (PC1) pour lequel les unités monomériques issues d'un monomère neutre sont choisies parmi le 2-hydroxyétyl acrylate ou le 2-hydroxyéthyl méthacrylate. De préférence, le monomère neutre est le 2-hydroxyétyl acrylate.
- le polyélectrolyte cationique réticulé (PC1) est un polyélectrolyte cationique réticulé (PC1) pour lequel dans ledit monomère de formule (VI), R représente un radical alkyle comportant de 12 à 18 atomes de carbone, et encore plus particulièrement R représente un radical alkyle choisi parmi les éléments du groupe constitué par le radical dodécyle, tridécyle, tétradécyle, hexadécyle, octadécyle.

- le polyélectrolyte cationique réticulé (PC1) est un polyélectrolyte cationique réticulé (PC1) pour lequel dans ledit monomère de formule (VI), n représente un nombre entier supérieur ou égal à 3 et inférieur ou égal à 20.
- le polyélectrolyte cationique réticulé (PC1) est un polyélectrolyte cationique réticulé (PC1) pour lequel ledit monomère de formule (VI) est le méthacrylate de lauryle tétraéthoxylé ou le méthacrylate de stéaryle eicosaéthoxylé.
- le polyélectrolyte cationique réticulé (PC1) est choisi parmi les terpolymères du chlorure de N,N,N-triméthyl 3-[(1-oxo 2-propènyl) amino] propanammonium (APTAC), du 2-hydroxyéthyl acrylate et du méthacrylate de lauryle tétraéthoxylé, réticulé au triméthylol propanetriacrylate ou les terpolymères du chlorure de N,N,N-triméthyl 3-[(1-oxo 2-propènyl) amino] propanammonium (APTAC), du 2-hydroxyéthyl acrylate et du méthacrylate de stéaryle eicosaéthoxylé, réticulé au triméthylol propanetriacrylate ou au diméthacrylate d'éthylèneglycol ou au diacrylate d'éthylèneglycol.
- le polyélectrolyte cationique réticulé (PC) est un terpolymère de chlorure de N,N,N-triméthyl 3-[(1-oxo 2-propènyl) amino] propanammonium (APTAC), du 2-hydroxyéthyl acrylate et du méthacrylate de lauryle tétraéthoxylé, réticulé au triméthylol propanetriacrylate ou au diméthacrylate d'éthylèneglycol ou au diacrylate d'éthylèneglycol.
- le polyélectrolyte cationique réticulé (PC1) comporte pour 100% molaire :
   - de 45% molaire à 60% molaire d'unités monomériques issues du chlorure de N,N,N-triméthyl 3-[(1-oxo 2-propènyl) amino] propanammonium
   - de 0,1% molaire à 3% molaire d'unités monomériques issues du méthacrylate de lauryle tétraéthoxylé, et
   - de 35% molaire à 54,5% molaire d'unités monomériques issues du 2-hydroxyéthyl acrylate.

Le polyélectrolyte cationique réticulé (PC1) objet de la présente invention est préparé par la mise en oeuvre des procédés de polymérisation en solution, de polymérisation en suspension, de polymérisation en suspension inverse, de polymérisation en émulsion, de polymérisation en émulsion inverse, de polymérisation en milieu solvant suivie d'une étape de précipitation du polymère formé, tels que précédemment décrits pour la préparation du polyélectrolyte cationique réticulé (PC).

L'invention a pour aussi pour objet l'utilisation de la composition (C1) telle que définie précédemment, pour améliorer le peignage de mèches de cheveux.

Selon un aspect plus particulier, l'invention a pour objet l'utilisation de la composition (C1) telle que définie précédemment, pour améliorer le peignage des pointes de cheveux.

La composition (C1) objet de la présente invention telle que définie précédemment est destinée à être appliquée sur le cheveux et/ou le cuir chevelu de façon directe ou indirecte, comme par exemple par l'intermédiaire de tout type de support destiné à être mis en contact avec la peau (papier, lingette, textile, dispositif transdermique, etc.). Cette étape d'application de la composition (C1) selon l'invention intervient après au moins une étape de lavage desdits cheveux et suivie d'au moins une étape de rinçage ; cette étape d'application de la composition (C1) selon l'invention est suivie par une autre étape de rinçage, et optionnellement de séchage des cheveux ainsi lavés, traités et rincés.

La composition (C1) objet de la présente invention peut être conditionnée sous forme pressurisée dans un dispositif aérosol ou dans un dispositif de type « flacon pompe », dans un dispositif muni d'une paroi ajourée par exemple une grille, dans un dispositif muni d'un applicateur a billes (dit"roll-on).

La composition (C1) objet de la présente invention peut en outre comporter des excipients et/ou des principes actifs habituellement mis en oeuvre dans le domaine des formulations destinées à être appliquées sur le cheveu ou le cuir chevelu.

La composition (C1) telle que définie précédemment comprend en outre, un ou plusieurs composés auxiliaires choisis parmi les tensioactifs moussants et/ou détergents, les tensioactifs épaississants et/ou gélifiants, les agents épaississants et/ou gélifiants, les agents stabilisants, les composés filmogènes, les solvants et cosolvants, les agents hydrotropes, les agents plastifiants, les agents émulsionnants et co-émulsionnants, les agents opacificants, les agents nacrants, les agents surgraissants, les séquestrants, les agents chélatants, les agents antioxydants, les parfums, les huiles essentielles, les agents conservateurs, les agents conditionneurs, les agents déodorants, les agents blanchissants destinés à la décoloration des poils et de la peau, les principes actifs destinés à apporter une action traitante et/ou protectrice vis à vis de la peau ou des cheveux, les filtres solaires, les charges minérales ou les pigments, les particules procurant un effet visuel ou destinées à l'encapsulation d'actifs, les particules exfoliantes, les agents de texture, les azurants optiques, les répulsifs pour les insectes.

Comme exemples de tensioactifs moussants et/ou détergents, éventuellement présents dans la composition cosmétique (C1) objet de la présente invention, on peut citer les tensioactifs moussants et/ou détergents anioniques, cationiques, amphotères ou non ioniques topiquement acceptables habituellement utilisés dans ce domaine d'activité.

Parmi les tensioactifs anioniques moussants et/ou détergents que l'on peut associer à la composition cosmétique (C1) objet de la présente invention, on peut citer les sels de métaux alcalins, les sels de métaux alcalino-terreux, les sels d'ammonium, les sels d'amines, les sels d'amino alcools d'alkyléthers sulfates, d'alkyl sulfates, d'alkylamidoéther sulfates, d'alkylarylpolyéther sulfates, de monoglycérides sulfates, d'alpha-oléfinesulfonates, de paraffines sulfonates, d'alkyl phosphates, d'alkyléther phosphates, d'alkyl sulfonates, d'alkylamide sulfonates, d'alkylaryl sulfonates, d'alkyl carboxylates, d'alkylsulfosuccinates, d'alkyléther sulfosuccinates, d'alkylamide sulfosuccinates, d'alkyl sulfo-acétates, d'alkyl sarcosinates, d'acyliséthionates, de N-acyl taurates, d'acyl lactylates, de dérivés N-acylés d'acides aminés, de dérivés N-acylés de peptides, de dérivés N-acylés de protéines, d'acides gras.

Parmi les tensioactifs amphotères moussants et/ou détergents éventuellement présents dans la composition cosmétique (C1) objet de la présente invention, on peut citer les alkylbétaines, les alkylamidobétaines, les sultaines, les alkylamidoalkylsulfobétaines, les dérivés d'imidazolines, les phosphobétaïnes, les amphopolyacétates et les amphopropionates.

Parmi les tensioactifs cationiques moussants et/ou détergents éventuellement présents dans la composition cosmétique (C1) objet de la présente invention, on peut citer particulièrement les dérivés d'ammoniums quaternaires.

Parmi les tensioactifs non ioniques moussants et/ou détergents éventuellement présents dans la composition cosmétique (C1) objet de la présente invention, on peut citer plus particulièrement les alkylpolyglycosides comportant un radical aliphatique, linéaire ou ramifié, saturé ou insaturé, et comportant de 8 à 12 atomes de carbone ; les dérivés d'huile de ricin, les polysorbates, les amides de coprah, les N-alkylamines.

Comme exemples de tensioactifs épaississants et/ou gélifiants éventuellement présents dans la composition cosmétique (C1) objet de la présente invention, on peut citer :
- les esters gras d'alkylpolyglycosides éventuellement alcoxylés, et tout particulièrement les esters de méthylpolyglucoside éthoxylés tels que le PEG 120 méthyl glucose trioléate et le PEG 120 méthyl glucose dioléate commercialisés respectivement sous les appellations GLUCAMATE^{™} LT et GLUMATE^{™} DOE120 ;
- les esters gras alcoxylés tels que le PEG 150 pentaérythrytyl tétrastéarate commercialisé sous l'appellation CROTHIX^{™} DS53, le PEG 55 propylene glycol oléate commercialisé sous l'appellation ANTIL^{™} 141 ;
- les carbamates de polyalkylène glycols à chaînes grasses tels que le PPG 14 laureth isophoryl dicarbamate commercialisé sous l'appellation ELFACOS^{™} T211, le PPG 14 palmeth 60 hexyl dicarbamate commercialisé sous l'appellation ELFACOS^{™} GT2125.

Comme exemples de tensioactifs émulsionnants éventuellement présents dans la composition cosmétique (C1) objet de la présente invention, on peut citer des tensioactifs non ioniques, des tensioactifs anioniques, des tensioactifs cationiques.

Comme exemples de tensioactifs non ioniques émulsionnants éventuellement présents dans la composition cosmétique (C1) objet de la présente invention, on peut citer les esters d'acides gras et de sorbitol, par exemple les produits commercialisés sous les appellations MONTANE^{™}80 et MONTANE^{™}85 et MONTANE^{™}60; l'huile de ricin éthoxylée et l'huile de ricin hydrogénée éthoxylée, par exemple le produit commercialisé sous la dénomination SIMULSOL^{™} 989; les compositions comprenant du stéarate de glycérol et de d'acide stéarique poly(éthoxylé) avec entre 5 moles et 150 moles d'oxyde d'éthylène, par exemple la composition comprenant de l'acide stéarique (éthoxylé) à 135 moles d'oxyde d'éthylène et du stéarate de glycérol commercialisée sous l'appellation SIMULSOL^{™} 165 ; les esters de sorbitan éthoxylés, par exemple les produits commercialisés sous la dénomination MONTANOX^{™} ; les esters de mannitan ; les esters de mannitan éthoxylés ; les esters de sucrose ; les esters de méthylglucoside.

Comme exemples de tensioactifs anioniques émulsionnants éventuellement présents dans la composition cosmétique (C1) objet de la présente invention, on peut citer le décylphosphate, le cétylphosphate commercialisé sous l'appellation AMPHISOL^{™}, le glycéryl stéarate citrate ; le cétéarylsulfate ; la composition arachidyl/béhényl phosphates et arachidyl/béhényl alcools commercialisée sous l'appellation SENSANOV^{™}WR; les savons par exemple le stéarate de sodium ou le stéarate de triéthanolammonium, les dérivés N-acylés d'acides aminés salifiés comme par exemple le stéaroyl glutamate.

Comme exemples de tensioactifs cationiques émulsionnants éventuellement présents dans la composition cosmétique (C1) objet de la présente invention, on peut citer les aminoxydes, le quaternium-82 et les tensioactifs décrits dans la demande de brevet WO96/00719 et principalement ceux dont la chaîne grasse comprend au moins 16 atomes de carbone.

Comme exemples d'agents opacifiants et/ou nacrants éventuellement présents dans la composition cosmétique (C1) objet de la présente invention, on peut citer le palmitate de sodium, le stéarate de sodium, l'hydroxystéarate de sodium, le palmitate de magnésium, le stéarate de magnésium, l'hydroxystéarate de magnésium, le monostéarate d'éthylène glycol, le distéarate d'éthylène glycol, le monostéarate de polyéthylène glycol, le distéarate de polyéthylène glycol, les alcools gras comportant de 12 à 22 atomes de carbone.

Comme exemples d'agents de texture éventuellement présents dans la composition cosmétique (C1) objet de la présente invention, on peut citer des dérivés N-acylés d'acides aminés, par exemple la lauroyl lysine commercialisée sous l'appellation AMINOHOPE^{™}LL, l'octenyl starch succinate commercialisé sous l'appellation DRYFLO^{™}, le myristyl polyglucoside commercialisé sous l'appellation MONTANOV 14, les fibres de cellulose, les fibres de coton, les fibres de chitosane, le talc, la séricite, le mica.

Comme exemples de solvants et de co-solvants éventuellement présents dans la composition cosmétique (C1) objet de la présente invention, on peut citer l'eau, les solvants organiques par exemple le glycérol, le diglycérol, les oligomères du glycérol, l'éthylène glycol, le propylène glycol, le butylène glycol, l'hexylène glycol, le diéthylène glycol, le xylitol, l'érythritol, le sorbitol, les alcools hydrosolubles tels que l'éthanol, l'isopropanol ou le butanol, les mélanges d'eau et desdits solvants organiques.

Comme exemples de principes actifs éventuellement présents dans la composition cosmétique (C1) objet de la présente invention, il y a :
- Les vitamines et leurs dérivés, par exemple, le rétinol (vitamine A) et ses esters (palmitate de rétinyle par exemple), l'acide ascorbique (vitamine C) et ses esters, les dérives de sucre de l'acide ascorbique (par exemple l'ascorbyl glucoside), le tocophérol (vitamine E) et ses esters (par exemple l'acétate de tocophérol), les vitamines B3 ou B10 (niacinamide et ses dérivés) ;
- Les composés ayant une action éclaircissante ou dépigmentante de la peau, par exemple le SEPIWHITE^{™}MSH, l'arbutine, l'acide kojique, l'hydroquinone, le VEGEWHITE^{™}, la GATULINE^{™}, le SYNERLIGHT^{™}, le BIOWHITE^{™}, le PHYTOLIGHT^{™}, le DERMALIGHT^{™}, la CLARISKIN^{™}, le MELASLOW^{™}, le DERMAWHITE^{™}, l'ETHIOLINE, le MELAREST^{™}, le GIGAWHITE^{™}, l'ALBATINE^{™}, le LUMISKIN^{™} ;
- Les composés ayant une action apaisante comme le SEPICALM^{™} S, l'allantoïne et le bisabolol ;
- Les agents anti-inflammatoires ;
- Les composés montrant une action hydratante par exemple l'urée, les hydroxyurées, le glycérol, les polyglycérols, le glycérolglucoside, le diglycérolglucoside, les polyglycérylglucosides ;
- Les composés montrant une action amincissante ou lipolytique comme la caféine ou ses dérivés, l'ADIPOSLIM^{™}, l'ADIPOLESS^{™} ;
- Les protéines N-acylées ; les peptides N-acylés par exemple le MATRIXIL^{™} ; les acides aminés N-acylés ; les hydrolysâts partiels de protéines N-acylés ; les acides aminés ; les peptides ; les hydrolysâts totaux de protéines ;
- Les extraits végétaux riches en tanins en polyphénols et/ ou en isoflavones, par exemple les extraits de raisin, les extraits de pin, les extraits de vin, les extraits d'olives ; les extraits de soja, par exemple la Raffermine^{™} ; les extraits de blé par exemple la TENSINE^{™} ou la GLIADINE^{™} ; les extraits végétaux riches en terpènes ; les extraits d'algues d'eau douce ou marines ; les extraits marins en général comme les coraux ;
- Les cires essentielles ; les extraits bactériens ; les céramides ou les phospholipides ;
- Les composés ayant une action antimicrobienne ou une action purifiante, par exemple le LIPACIDE^{™} C8G, le LIPACIDE^{™} UG, le SEPICONTROL^{™} A5 ; l'OCTOPIROX^{™} ou le SENSIVA^{™} SC50 ;
- Les composés ayant une propriété énergisante ou stimulante comme le Physiogényl^{™}, le panthénol et ses dérivés comme le SEPICAP^{™} MP ;
- Les actifs anti-âge comme le SEPILIFT^{™} DPHP, le LIPACIDE^{™} PVB, le SEPIVINOL^{™}, le SEPIVITAL^{™}, le MANOLIVA^{™}, le PHYTO-AGE^{™}, le TIMECODE^{™} ; le SURVICODE^{™} ;
- Les actifs anti-photo vieillissement ; les actifs protecteurs de l'intégrité de la jonction dermo-épidermique ;
- Les actifs augmentant la synthèse des composants de la matrice extracellulaire par exemple le collagène, les élastines, les glycosaminoglycanes ;
- Les actifs agissant favorablement sur la communication cellulaire chimique comme les cytokines ou physiques comme les intégrines ;
- Les actifs créant une sensation de « chauffe » sur la peau comme les activateurs de la microcirculation cutanée (par exemple les dérivés de l'acide nicotinique) ou des produits créant une sensation de « fraîcheur » sur la peau (par exemple le menthol et des dérivés) ;

Les actifs améliorant la microcirculation cutanée, par exemple les veinotoniques ; les actifs drainants ; les actifs à visée décongestionnante par exemple les extraits de ginko biloba, de lierre, de marron d'inde, de bambou, de ruscus, de petit houx, de *centalla asiatica,* de fucus, de romarin, de saule ;
- Les agents de bronzage ou de brunissement de la peau, par exemple la dihydroxyacétone, l'isatine, l'alloxane, la ninhydrine, le glycéraldéhyde, l'aldéhyde mésotartrique, la glutaraldéhyde, l'érythrulose.

Comme exemples d'agents épaississants et/ou gélifiants éventuellement présents dans la composition cosmétique (C1) selon l'invention, on peut citer les polysaccharides constitués uniquement d'oses, comme les glucanes ou homopolymères du glucose, les glucomannoglucanes, les xyloglycanes, les galactomannanes dont le degré de substitution (DS) des unités de D-galactose sur la chaîne principale de D-mannose est compris entre 0 et 1, et plus particulièrement entre 1 et 0,25, comme les galactomannanes provenant de la gomme de cassia (DS = 1/5), de la gomme de caroube (DS = 1/4), de la gomme de tara (DS = 1/3), de la gomme de guar (DS = 1/2), de la gomme de fenugrec (DS = 1).

Comme exemples d'agents épaississants et/ou gélifiants éventuellement présents dans la composition cosmétique (C1) selon l'invention, on peut citer les polysaccharides constitués de dérivés d'oses, comme les galactanes sulfatés et plus particulièrement les carraghénanes et l'agar, les uronanes et plus particulièrement les algines, les alginates et les pectines, les hétéropolymères d'oses et d'acides uroniques et plus particulièrement la gomme xanthane, la gomme gellane, les exsudats de gomme de arabique et de gomme de karaya, les glucosaminoglycanes.

Comme exemples d'agents épaississants et/ou gélifiants éventuellement présents dans la composition cosmétique (C1) selon l'invention, on peut citer la cellulose, les dérivés de cellulose comme la méthyl-cellulose, l'éthyl-cellulose, l'hydroxypropyl cellulose, les silicates, l'amidon, les dérivés hydrophiles de l'amidon, les polyuréthanes.

Comme exemples d'agents stabilisants éventuellement présents dans la composition cosmétique (C1) selon l'invention, on peut citer les cires microcristallines, et plus particulièrement l'ozokérite, les sels minéraux tels que le chlorure de sodium ou le chlorure de magnésium.

Comme exemples d'eaux thermales ou minérales que l'on peut associer à la composition selon l'invention, on peut citer les eaux thermales ou minérales ayant une minéralisation d'au moins 300 mg/l, en particulier l'eau d'Avene, l'eau de Vittel, les eaux du bassin de Vichy, l'eau d'Uriage, l'eau de la Roche Posay, l'eau de la Bourboule, l'eau d'Enghien-les-bains, l'eau de Saint-Gervais-les bains, l'eau de Néris-les-bains, l'eau d'Allevard-les-bains, l'eau de Digne, l'eau des Maizieres, l'eau de Neyrac-les-bains, l'eau de Lons le Saunier, l'eau de Rochefort, l'eau de Saint Christau, l'eau des Fumades et l'eau de Tercis-les-bains.

La composition cosmétique (C1) selon l'invention et telle que définie précédemment, est obtenue par la mise en oeuvre du procédé de préparation comprenant les étapes suivantes :
Une étape a) de préparation de la phase grasse (A₂) en mélangeant l'ensemble des éléments la constituant dans les proportions souhaitées. Cette étape de mélange est généralement conduite à une température supérieure ou égale à 20°C et inférieure ou égale à 80°C, plus particulièrement supérieure ou égale à 25°C et inférieure ou égale à 80°C, et encore plus particulièrement supérieure ou égale à 30°C et inférieure ou égale à 80°C ; elle est réalisée sous agitation mécanique à une vitesse modérée supérieure ou égale à 50 tours/minute et inférieure ou égale à 100 tours/minute ;
Une étape b) de préparation de la phase aqueuse (A₁) de l'ensemble des éléments la constituant dans les proportions souhaitées. Cette étape de mélange est généralement conduite à une température supérieure ou égale à 20°C et inférieure ou égale à 80°C, plus particulièrement supérieure ou égale à 20°C et inférieure ou égale à 60°C, et encore plus particulièrement supérieure ou égale à 20°C et inférieure ou égale à 40°C ; elle est réalisée sous agitation mécanique à une vitesse modérée supérieure ou égale à 500 tours/minute et inférieure ou égale à 3 000 tours/minute. De façon particulière, la phase aqueuse (A₁) obtenue à l'issue de l'étape b), présente une viscosité dynamique, mesurée à 20°C par l'intermédiaire d'un viscosimètre de type Brookfield LV à une vitesse de 6 tours/minute, supérieure ou égale à 200 mPa.s et inférieure ou égale à 40 000 mPa.s, plus particulièrement supérieure ou égale à 1 000 mPa.s et inférieure ou égale à 40 000 mPa.s, et encore plus particulièrement supérieure ou égale à 2 000 mPa.s et inférieure ou égale à 40 000 mPa.s ;
Une étape c) au cours de laquelle la phase grasse (A₂) est ajoutée sur la phase aqueuse (A₁) à une température supérieure ou égale à 20°C et inférieure ou égale à 80°C, plus particulièrement supérieure ou égale à 20°C et inférieure ou égale à 60°C, et encore plus particulièrement supérieure ou égale à 20°C et inférieure ou égale à 40°C, sous agitation mécanique à une vitesse modérée supérieure ou égale à 50 tours/minute et inférieure ou égale à 400 tours/minute, de façon à obtenir la composition selon l'invention.

Les exemples suivants illustrent l'invention, sans toutefois la limiter.

### Préparation et évaluation d'émulsions eau-dans-huile selon l'invention et d'émulsions eau-dans-huile comparatives.

### I) - Préparation de polyélectrolytes cationiques réticulés.

I-1 On prépare un latex inverse auto-inversible du copolvmère APTAC/HEA/MAL(40E) (49.76/49.76/0.48) réticulé au MBA comme suit :
On charge dans un bêcher, sous agitation: 151.0 g d'eau permutée ; 147,9 g d'hydroxyéthyl acrylate ; 351,0 g d'une solution commerciale à 75% de chlorure d'acrylamido-propyltriméthylammonium (APTAC) ; 0,224 g d'éthylène glycol di méthacrylate ; 0,45 g d'une solution aqueuse commerciale à 40% du sel de sodium de l'acide diéthylènetriamine pentaacétique.

On prépare une phase huile en mélangeant successivement : 259 g d'isohexadécane 20 g d'isostéarate de sorbitan (commercialisé sous le nom de marque MONTANE^{™} 70 par la société SEPPIC) 5 g d'HYPERMER^{™} 2296 (commericalisé par la société Croda) 5 g de méthacrylate de lauryle tétraéthoxylé [MAL(40E)] 0.1 g d'azo-bis(isobutyronitrile) (AIBN).

La phase aqueuse est incorporée progressivement dans la phase organique puis soumise à une agitation mécanique violente au moyen d'une turbine de type ULTRA-TURRAX^{™}, pour former une émulsion inverse (eau/huile).

L'émulsion est ensuite refroidie jusqu'à environ 10°C et placée sous barbotage d'azote pendant environ 60 minutes pour en éliminer l'oxygène. La polymérisation est alors initiée en y incorporant 10 cm<3>d'une solution d'hydroperoxyde de cumène à 0,68 % en poids dans l'isohexadecane. Après homogénéisation du milieu, on ajoute 25 g d'une solution aqueuse de métabisulfite de sodium à 0,1 % en poids en laissant monter la température du mélange jusqu'à la température finale de polymérisation puis en laissant le mélange pendant 90 minutes. L'ensemble est ensuite refroidi jusqu'à 35° C environ, puis on ajoute 40 g d'alcool laurique éthoxylé à 7 moles (SIMULSOL^{™}P7). On obtient le latex inverse auto inversible désiré (LI1).

### Analyses

### Teneur en polyélectrolyte: environ 27,5 % poids

### Mesures de viscosité :

- Viscosité du latex inverse auto inversible (LI1) (Brookfield RVT, Mobile 3; Vitesse: 5 tours par minute) : η = 1 280 mPa.s
- Viscosité d'une solution aqueuse à 3 % en poids du latex inverse auto-inversible (LI1) (Brookfield RVT, Mobile 6: ; Vitesse: 5 tours par minute) : η = 68 400 mPa.s
- Viscosité d'une solution aqueuse à 3% en poids du latex inverse auto-inversible (LI1) et à 1 %o de chlorure de sodium : η = 14 440 mPa.s.

### II) - Préparation et évaluation d'émulsions eau-dans-huile selon l'invention et d'émulsions eau-dans-huile comparatives.

### II-1 Préparation d'émulsions eau-dans-huile selon l'invention

On prépare une émulsion eau-dans-huile selon l'invention, notée (F₁), dont les proportions massiques de ses constituants sont consignées dans le tableau 1 ci-dessous, les teneurs massiques des polyélectrolytes étant indiquées en pourcentage de matière sèche polymérique, en mettant en oeuvre le procédé suivant :
Les constituants de la phase grasse sont introduits successivement dans un bêcher, et mélangés à une température de 20°C, à l'aide d'un agitateur mécanique muni d'un mobile d'agitation de type hélice, à une vitesse de 100 tours/minute. La glycérine et l'eau sont mélangées à température ambiante dans un bêcher à l'aide d'un agitateur mécanique à une vitesse de 2 000 tours/minute et l'agent épaississant est alors ajouté progressivement.

L'agitation est maintenue pendant une durée permettant d'atteindre une phase aqueuse se présentant sous la forme d'un gel homogène. La phase grasse est ajoutée en une seule fois sur le gel aqueux, à température ambiante et à une vitesse d'agitation modérée (75 à 300 tours/minute) avec un agitateur équipé d'un mobile de type ancre. Cette agitation est alors maintenue pendant dix minutes et aucune étape de refroidissement n'est nécessaire.

La conductivité **(σ)** des émulsions comparatives (F'₁) à (F'₃) est mesurée à 20°C, après une durée de stockage desdites émulsions de un jour dans une enceinte climatique isolée et régulée à une température de 20°C, au moyen d'un conductimètre de marque LF 196^{™} de la société WTW muni d'une électrode Tétracon^{™} 96.

Lorsque **(σ)** ≤ 0,5 µS.cm⁻¹, on considère que l'émulsion n'est pas conductrice et que par conséquent la phase externe n'est pas la phase aqueuse mais la phase huileuse,et que ladite émulsion est de type eau-dans-huile.

**Tableau 1**

| | **(F1)** |
|---|---|
| **Phase grasse** | |
| Huile d'olive ⁽¹⁾ | 1% |
| Triglycérides 5545 ⁽²⁾ | 9% |
| Euxyl^{™} PE9010 ⁽³⁾ | 1% |
| Easynov^{™ (4)} | 2,5% |
| **Phase aqueuse** | |
| Glycérine | 2% |
| Latex inverse (LI1) | 0,6% |
| | (m.a.p)* |
| | soit 2,18 % |
| | de (LI1) |
| SIMULGEL^{™} 600⁽⁵⁾ | 0% |
| Eau | Qs 100% |
| Ajustement pH | Qs |
| | pH = 5,0 |
| **(σ)** à 1 jour | ≤ 0,5 |
| | µS.cm⁻¹ |

| | |
|---|---|
| (1): Huile d'olive (nom INCI : *Olea Europea (Olive) Fruit Oil*) : huile utilisée comme phase huileuse dans la préparation de composition cosmétique; (2) : Triglycérides 5545 (nom INCI : *Caprylic*/*Capric Triglycérides)* : composition utilisée comme phase huileuse dans la préparation de composition cosmétique; (3) : EUXYL^{™} PE9010 (Nom INCI : Phenoxyethanol & Ethylhexylglycerin) : Composition utilisée comme agent conservateur. (4) : EASYNOV^{™} (nom INCI : Octyldodecanol, Octyldodecyl Xyloside and PEG-30 Dipolyhydroxystearate) : composition émulsionnante commercialisée par la société SEPPIC, comprenant pour 100% de sa masse, de 55% à 65% massique de 2-Octyldodécanol, de 15% à 25% massique de 2-Octyldodécylpolyxyloside, de 10 % à 30 % massique de PEG-30 Dipolyhydroxystearate ; (5) : SIMULGEL^{™} 600 (Nom INCI : acrylamide/sodium acryloyldimethyltaurate copolymer and isohexadecane and polysorbate 80) : Latex inverse comprenant environ pour 100% de sa masse, environ 35% massique d'un copolymère de l'acide acrylique partiellement salifié et du sel de sodium de l'acide 2-méthyl 2-[(1-oxo 2-propènyl) amino] 1-propanesulfonique, réticulé au méthylène-bis-acrylamide ; (*) : m.a.p : matière active polylmérique. | |

### II-2 Préparation d'émulsions eau-dans-huile et huile-dans-eau comparatives

a) On prépare une émulsion eau-dans-huile comparative selon le procédé décrit dans le paragraphe II-1, notée (F'₁), et dont les proportions massiques de ses constituants sont consignées dans le tableau 2 ci-dessous.
b) On prépare une émulsion eau-dans-huile comparative notée (F'₂), dont les proportions massiques de ses constituants sont consignées dans le tableau 2 ci-dessous, selon le procédé suivant :
   - les constituants de la phase grasse sont introduits successivement dans un bêcher, et mélangés à une température de 85°C, à l'aide d'un agitateur mécanique muni d'un mobile d'agitation de type hélice, à une vitesse de 100 tours/minute ;
   - l'eau chauffée à 85°C est ajoutée sur la phase grasse à 85°C, puis le sulfate de magnésium et la glycérine sont ajoutés.
   - Le mélange obtenu est maintenu à 85°C sous agitation mécanique de type hélice, à une vitesse de 100 tours/minute ;
   - Le mélange est ensuite agité avec un dispositif mécanique de type rotor-stator de marque Silverson, à une vitesse de 2 000 tours.min⁻¹ pendant deux minutes, puis de 8 000 tours.min⁻¹ pendant deux minutes ;
   - Le mélange ainsi homogénisé est alors maintenu sous agitation mécanique de type hélice, à une vitesse de 200 tours/minute en refroidissant jusqu'à 20°C, puis vidangée pour obtenir l'émulsion eau-dans-huile (F'₂).
c) On prépare une émulsion huile-dans-eau comparative notée (F'₃), dont les proportions massiques de ses constituants sont consignées dans le tableau 2 ci-dessous, selon le procédé suivant :
   - les constituants de la phase grasse sont introduits successivement dans un bêcher, et mélangés à une température de 75°C, à l'aide d'un agitateur mécanique muni d'un mobile d'agitation de type hélice, à une vitesse de 100 tours/minute ;
   - l'eau chauffée à 75°C est ajoutée sur la phase grasse à 75°C, puis la glycérine est ajoutée.
   - Le latex inverse (LI1) est alors ajouté à 75°C, puis le mélange résultant est homogénisé avec un dispositif mécanique de type rotor-stator de marque Silverson, à une vitesse de 4 000 tours.min⁻¹ pendant quatre minutes,
   - Le mélange ainsi homogénisé est alors maintenu sous agitation mécanique de type hélice, à une vitesse de 100 tours/minute pendant 10 minutes en refroidissant jusqu'à 20°C, puis vidangée pour obtenir l'émulsion eau-dans-huile (F'₃).
d) La conductivité **(σ)** des émulsions comparatives (F'₁) à (F'₃) est mesurée à 20°C, après une durée de stockage desdites émulsions de un jour dans une enceinte climatique isolée et régulée à une température de 20°C, au moyen d'un conductimètre de marque LF 196^{™} de la société WTW muni d'une électrode Tétracon^{™} 96.

Lorsque **(σ)** ≤ 0,5 µS.cm⁻¹, on considère que l'émulsion n'est pas conductrice et que par conséquent la phase externe n'est pas la phase aqueuse mais la phase huileuse,et que ladite émulsion est de type eau-dans-huile.

Lorsque **(σ)>** 0,5 µS.cm⁻¹, on considère que l'émulsion est conductrice et que par conséquent la phase externe est la phase aqueuse et que ladite émulsion est de type huile-dans-eau.

**Tableau 2**

| | **(F'₁)** | **(F'₂)** | **(F'₃)** |
|---|---|---|---|
| **Phase grasse** | | | |
| Huile d'olive⁽¹⁾ | 1% | 1% | 1% |
| Triglycérides 5545⁽²⁾ | 9% | 12% | 9% |
| Euxyl^{™} PE9010⁽³⁾ | 1% | 1% | 1% |
| Easynov^{™(4)} | 2,5% | 4% | 0% |
| Simulsol^{™}165⁽⁶⁾ | 0% | 0% | 0% |

| **Phase aqueuse** | | | |
|---|---|---|---|
| Glycérine | 2% | 2% | 2% |
| SIMULGEL^{™} 600⁽⁵⁾ | 0,6% (m.a.p)* | 0% | 0% |
| | soit 1,71 % de polyélectrolyte anionique réticulé | | |
| Latex inverse (LI1) | 0% | 0% | 0,6% (m.a.p)* |
| | | | soit 2,18 % de |
| | | | (LI1) |
| MgSO₄, 7 H₂O | 0% | 0,72% | 0% |
| Eau | Qs 100% | Qs 100% | Qs 100% |
| Ajustement pH | Qs | Qs | Qs |
| | pH = 5,0 | pH = 5,0 | pH = 5,0 |
| **(σ)** à 1 jour | < 0,5 µS.cm⁻¹ | ≤ 0,5 µS.cm⁻¹ | > 0,5 µS.cm⁻¹ |

| | | | |
|---|---|---|---|
| (6) : Simulsol^{™}165 : Nom INCI PEG-100 stérate & glycéryl stéarate, est une composition utilisée comme agent tensioactif émulsionnant pour préparer des émulsions de type huile-dans-eau. | | | |

### II-3 Evaluation de l'émulsion et des émulsions eau-dans-huile et huile-dans-eau comparatives comme sur le peignage de mèches de cheveux

### II-3.1 Description de la méthode d'évaluation du peignage des mèches de cheveux

### a) Préparation des mèches de cheveux.

- On utilise des mèches de cheveux caucasiens abîmés de type « 4h » fournis par la société Kerling International, se présentant chacune sous la forme d'une mèche de 2 grammes [« glued weft 4H bleached, 20cm x 2cm / 2g colour 7/0 »].
- Pour chaque mèche, la partie supérieure des cheveux qui restent joints (maintien des cheveux entre eux), est recouverte d'un adhésif orange pour permettre le marquage du code. Chaque mèche est ensuite identifiée à l'aide d'un marqueur indélébile en inscrivant les deux derniers chiffres de l'année en cours, puis n° de la semaine, suivi de C et d'un numéro de 01 à 99 (ex : 1416C08).
- Au total, 3 mèches sont évaluées pour chaque émulsion à évaluer.

### b) Protocole d'essai

- Chaque mèche de cheveu est mouillée sous un filet d'eau, et l'excédent d'eau est enlevé en plaçant deux doigts de part et d'autre de la mèche en exerçant un mouvement vertical descendant.
- la mèche est trempée dans une solution de lavage, consistant en une solution à 10% en matière active de Lauryl éther sulfate de sodium à 2,2 moles d'oxyde d'éthylène, une fois ; l'excédent de solution de lavage est enlevé en plaçant deux doigts de part et d'autre de la mèche en exerçant un mouvement vertical descendant.
- La mèche est ensuite shampooinée pendant trente secondes en zig-zag. Ainsi, la mèche est posée dans le creux de la main directrice tout en maintenant la mèche par le haut avec l'autre main. On applique alors avec le pouce de la main directrice un mouvement horizontal de va et vient tout en tirant lentement la mèche avec l'autre main. Le pouce effectue alors un mouvement de zigzag le long de la mèche. On remonte en haut de la mèche entre chaque passage et il est nécessaire d'effectuer cette manipulation des deux côtés de la mèches (15 secondes pour chaque côté).
- l'opérateur doit se rincer les mains pour se débarrasser de toute trace de solution de lavage avant de poursuivre le protocole expérimental
- la mèche est ensuite rincée sous le robinet d'eau pendant trente secondes : quinze seconde de chaque côté en accompagnant deux fois de suite le filet d'eau avec les doigts.
- l'excédent d'eau est enlevé en plaçant deux doigts de part et d'autre de la mèche en exerçant un mouvement vertical descendant.
- Maintenir la mèche à la verticale au-dessus de la paillasse, et à l'aide de la partie «fine» du peigne, peigner la mèche 10 fois de chaque côté.
- Vérifier qu'il ne reste pas de tensio-actif sur la mèche
- la force nécessaire pour peigner chaque mèche est ensuite évaluée par l'intermédiaire de l'appareil Diastron MT175 (voir ci-dessous), par la réalisation de six mesures consécutives pour chaque mèche ; les valeurs obtenues constituent les valeurs « témoins ».
- chaque mèche est maintenue « en main » en la maintenant par le scotch et on applique 0.2 gramme d'émulsion à tester par gramme de cheveu sur le tiers supérieur de la mèche ;
- la mèche est alors shampooinée pendant trente secondes en zigzag de la même manière que pour le lavage
- L'opérateur se rince alors les mains pour se débarrasser de toute trace de produit
- la mèche est rincée sous le robinet d'eau pendant trente secondes : : quinze seconde de chaque côté en accompagnant deux fois de suite le filet d'eau avec les doigts.
- l'excédent d'eau est enlevé en plaçant deux doigts de part et d'autre de la mèche en exerçant un mouvement vertical descendant
- la mèche est maintenue verticalement et peignée dix fois de chaque côté avec la partie « fine » du peigne,
- la force nécessaire pour peigner chaque mèche est ensuite évaluée par l'intermédiaire de l'appareil Diastron MT175 (voir ci-dessous), par la réalisation de six mesures consécutives pour chaque mèche ; les valeurs obtenues constituent les valeurs « expérimentales » pour chaque émulsion testée.

### c) Mesures des forces nécessaires au peignage de mèches

### Matériel :

On utilise un appareil permettant de mesurer des forces de peignage de mèches de cheveux nommé « miniature tensile tester », modèle MTT 175, commercialisé par la société Diastron, équipé d'un logiciel UvWin (logiciel de gestion du MTT 175, d'acquisition et de traitement des données) et d'une unité de contrôle entre le MTT 175 et le logiciel UvWin nommée UV 1000.

Les peignes utilisés sont des peignes commercialisés par la société Babyliss, de la gamme Babyliss Pro Styling Comb (Référence 691078).

Les mèches utilisées sont des mèches de cheveux caucasiens abimés de type « 4h » fournis par la société Kerling International,

### Méthode de mesure :

- L'appareil Diastron MTT 175 permet de mesurer la force nécessaire pour peigner une mèche de cheveux.
- La méthode consiste à fixer la mèche à évaluer au dispositif ad-hoc de l'appareil, qui est aussi équipé d'un peigne fixé sur une partie mobile ; la mèche est fixée en position verticale
- Lorsqu'un mécanisme est activé, le peigne parcours la mèche en descendant le long de la mèche et à chaque point du parcours, l'appareil enregistre la force nécessaire pour poursuivre sa progression. Ainsi, l'appareil peut tracer l'évolution de la force de peignage en fonction de la longueur parcourue par le peigne le long de la mèche
- Ce protocole est réalisé six fois pour la même mèche : trois mesures pour chaque côté de la mèche.
- Au total, pour chaque mèche, on réalise six mesures de contrôle ou six mesures « témoins », à savoir sur la mèche lavée mais non traitées par l'émulsion à évaluer et six mesures sur la mèche lavée et traitée par l'émulsion à évaluer.
- Pour chaque mesure, il est donc possible d'obtenir l'évolution de la force

### Expression des résultats et interprétation :

La mesure réalisée permet de d'évaluer l'évolution de la force de friction ou force de peignage sur la longueur de la mèche parcourue par le peigne de la racine vers la pointe des cheveux.

Si la force de friction ou de peignage augmente au niveau de la pointe des cheveux, l'utilisateur aura l'impression d'un produit inefficace pour le démêlage, risquant même de s'arracher des cheveux et/ou de casser les pointes en forçant.

Le peignage idéal correspond à une faible force aussi constante que possible sur toute la longueur, de la racine à la pointe.

Pour chaque mesure, on retient la valeur de la force de peignage mesurée à 25% de la force maximale enregistrée, qui est exprimée en Newton (N).

On nommera ainsi la force de peignage de contrôle (Fcont), et la force de peignage pour le cheveu traité par les émulsions à évaluer (Fexp).

Si nécessaire, on peut également mesurer la valeur de la force de peignage en milieu de mèche et/ou en pointe de la mèche. Ainsi, on nommera (Fmil.) la valeur de la force de peignage en milieu de mèche (après 60,1 millimètre de peignage avec le dispositif utilisé) et (Fpoint) la valeur de la force de peignage en pointe de la mèche (soit après 159,2 millimètres de peignage avec le dispositif utilisé).

### II-3.2 Méthode d'évaluation de l'aspect et de la stabilité d'émulsions eau-dans-huile selon l'invention et d'émulsion comparatives

L'émulsions (F₁) selon l'invention, et les émulsions comparatives (F'₁) à (F'₃) sont conservées dans une enceinte climatique isolée et régulée à une température de 20°C pendant 7 jours. A l'issue de cette période de 7 jours, l'aspect (ASP) de chaque émulsion préparée est observé. Les émulsions sont ensuite replacées et conservées dans la même enceinte climatique isolée et régulée à une température de 20°C jusqu'à 3 mois. Après cette période, l'aspect (ASP) de chaque émulsion préparée est observé.

### 11-3.3 Résultats obtenus pour l'émulsion eau-dans-huile (F₁) selon l'invention et pour les émulsions comparatives (F'₁) à (F'₃)

### a) Résultats obtenus

Les méthodes d'évaluations décrites aux paragraphes II-3.1 et II-3.2 ont été appliquées à l'émulsion eau-dans-huile (F₁) selon l'invention et aux émulsions compratives (F'₁) à (F'₃). Les résultats obtenus sont consignés dans la tableau 3 ci-dessous.

**Tableau 3**

| | (F₁) | (F'₁) | (F'₂) | (F'₃) |
|---|---|---|---|---|
| **(ASP)** à 7 jours | Homogène | Homogène | Homogène | Homogène |
| **(ASP)** à 1 mois | Homogène | Homogène | Homogène | Homogène |
| **(ASP)** à 3 mois | Homogène | Homogène | Homogène | Homogène |
| **(Fcont.)** en Newton | 0,98 N | 1,26 N | 1,18 N | 1,16 N |
| **(Fexp.)** en Newton | 0,12 N | 0,49 N | 0,47 N | 0,16 N |
| **(Fmil)** en Newton | 11,8 N | n.m. | n.m. | 15,8 N |
| **(Fpoint)** en Newton | 13,1 N | n.m. | n.m. | 45,7 N |

| | | | | |
|---|---|---|---|---|
| n.m. : non mesuré. | | | | |

### b) Analyse des résultats obtenus

Le traitement des mèches de cheveux mouillés par l'émulsion (F₁) selon l'invention, permet de diminuer la force peignage de 87,8% entre les mèches non traitées (0,98 N) et les mèches traitées avec l'émulsion (F₁) (0,12 N), alors que l'émulsion comparative (F'₁), qui ne se distingue de l'émulsion (F₁) que de par la nature du polyélectrolyte réticulé utilisé, ne permet une diminution de la force de peignage de 61,1% (force de peignage de contrôle de 1,26 N et force de peignage des mèches traitées par (F'₁) de 0,49 N).

Il faut également noter que l'émulsion comparative eau-dans-huile (F'₂), qui se distingue par l'absence de polyélectrolyte dans sa formulation, ne permet de réduire la force de peignage d'une valeur de 59,6% (force de peignage de contrôle de 1,18 N et force de peignage des mèches traitées par (F'₂) de 0,47 N).

Seule l'émulsion comparative (F'₂) de type huile-dans-eau permet une réduction de la force de peignage similaire à celle de l'émulsion (F₁) selon l'invention (diminution de 86,2%, pour une force de peignage de contrôle de 1,16 N et force de peignage des mèches traitées par (F'₃) de 0,16 N).

Cependant, le traitement des mèches de cheveux par l'émulsion (F₁) selon l'invention permet d'obtenir une moindre force de peignage aux pointes des mèches : 13,1 Newton pour les mèches de cheveux traitées par l'émulsion (F₁) contre 45,7 Newton pour les mèches de cheveux traitées par l'émulsion comparative (F'₂).

### III) - Formules illustratives

Dans les formulations suivantes, les pourcentages sont exprimés en poids de la formulation.

### III-1 crème couleur de protection

| | |
|---|---|
| Eau | qsp 100% |
| Glycérine | 2% |
| Latex inverse auto inversible (LI1) | 1,45% |
| TEA 6% Triethanolamine | qs pH 5,5 |
| Easynov^{™} | 2,50% |
| Huile de Coco | 5% |
| Triglycérides 5545 (caprylic / capric triglyceride) | 5% |
| Huile de coco | 5% |
| Inula^{™} HC | 1% |
| Parfum | 0.5% |
| Tocopherol | 0.1% |
| Dissolvine^{™} GL47 | 0.25% |
| Euxyl^{™} PE9010 (INCI :phenoxyethanol and ethylhexylglycerin) | 1% |

### III-2 crème nutritive pour cheveux ethniques de protection

| NOM produit | % |
|---|---|
| Eau | qsp 100% |
| Glycérine | 2% |
| Latex inverse auto inversible (LI1) | 1,45% |
| TEA 6% Triethanolamine | qs pH 5,5 |
| Easynov^{™} | 2,80% |
| Beurre de Karité | 4% |
| Huile d'argan | 2% |
| Emosmart^{™} V21 | 5% |
| Xylishine | 3% |
| Parfum | 0.5% |
| Tocopherol | 0.1% |
| Dissolvine GL47 | 0.25% |
| Euxyl^{™} PE9010 | 1% |

### III-3 sérum hydratant ultra brillant pour cheveux normaux

| NOM produit | % |
|---|---|
| Eau | qsp 100% |
| Glycérine | 2% |
| Latex inverse auto inversible (LI1) | 1,45% |
| TEA 6% Triethanolamine | qs pH 5,5 |
| Easynov^{™} | 2,0% |
| Caprylic / capric triglycéride | 2% |
| Huile d'argan | 1% |
| Emogreen^{™} L19 | 4% |
| Xylishine^{™} | 3% |
| Parfum | 0.5% |
| Tocopherol | 0.1% |
| Dissolvinet GL47 | 0.25% |
| Euxyl^{™} PE9010 | 1% |

### III-4 sérum crème botanique très doux

| NOM produit | % |
|---|---|
| Rosa damascena flower water | qsp 100% |
| Glycérine | 2% |
| Latex inverse auto inversible (LI1) | 1,45% |
| Triethanolamine 6% | qs pH 5,5 |
| Easynov^{™} | 2,6% |
| huile d'argan | 2% |
| Huile de coco | 1% |
| Emogreen^{™} L19 | 2% |
| Sodium Phytate | 0.1% |
| Tocopherol | 0.25% |
| Euxyl^{™} K712 sodium benzoate and potassium sorbate | 1.2% |

Easynov^{™} (INCI : octyldodecanol octyldodecyl xyloside and peg30 dipolyhydroxystearate) est un agent émulsionnant de type eau-dans-huile ;
Inula^{™} HC (INCI : caprylic/capric triglyceride- inula crithmoïde leaf/flower extract) est un ingrédient actif destine à protéger les cheveux des stresses extérieurs ;
Dissolvine^{™} GL47 (INCl :Tetrasodium Glutamate Diacetate) est un agent séquestrant ; Euxyl^{™} PE9010 (INCI :phenoxyethanol and ethylhexylglycerin) est un agent conservateur ; Emosmart^{™} V21 (INCI : C18-21 Alkane) est un agent émollient;
Emogreen^{™} L19 (INCI : C15-19 Alkane) est un agent émollient;
Xylishine^{™} (INCI name : Xylitylglucoside (and) Anhydroxylitol (and) Maltitol (and) Xylitol (and) Pelvetia Canaliculata Extract) est un agent actif destiné améliorer la brilliance des cheveux;
Euxyl^{™} K712 (INCI : sodium benzoate and potassium sorbate) est un agent conservateur

## Revendications

1. Composition cosmétique (C1) se présentant sous la forme d'une émulsion de type eau dans huile comprenant pour 100% de sa masse :
- de 60% à 95% massique d'une phase aqueuse gélifiée (A1) comprenant au moins un polyélectrolyte cationique réticulé qui comprend une proportion supérieure ou égale à 5% molaire d'unités monomériques issues d'un élément du groupe constitué par le chlorure de 2,N,N,N-tétraméthyl 2-[(1-oxo 2-propènyl) amino] propanammonium (AMPTAC), le chlorure de 2,N,N-triméthyl 2-[(1-oxo 2-propènyl) amino] propanammonium, le chlorure de N,N,N-triméthyl 3-[(1-oxo 2-propènyl) amino] propanammonium (APTAC),
- de 5% à 40% massique d'une phase grasse (A2) comprenant au moins une huile et un système émulsionnant comprenant une combinaison d'au moins un agent tensioactif émulsionnant (S) sélectionné parmi les éléments du groupe constitué par les compositions d'alkylpolyglycosides, les compositions d'alkylpolyglycosides et d'alcools gras, les esters de polyglycérols, les esters de polyglycérols alcoxylés, les polyhydroxystéarates de polyglycols, les polyhydroxystéarates de polyglycérols, les polyhydroxystéarates de polyglycérols alcoxylés. ».

2. Composition (C1) telle que définie dans la revendication 1 **caractérisée en ce que** la phase aqueuse gélifiée (A1) comprend pour 100% de sa masse de 0,1% à 7% massique d'un polyélectrolyte cationique réticulé (PC) et de 93% à 99,9% massique d'eau.

3. Composition (C1) telle que définie à l'une ou quelconque des revendications 1 ou 2 **caractérisée en ce que** le polyélectrolyte cationique réticulé (PC) comprend pour 100% molaire :
a1) de 5% molaire à 60% molaire d'unités monomériques issues d'un élément du groupe constitué par le chlorure de 2,N,N,N-tétraméthyl 2-[(1-oxo 2-propènyl) amino] propanammonium (AMPTAC), le chlorure de 2,N,N-triméthyl 2-[(1-oxo 2-propènyl) amino] propanammonium, le chlorure de N,N,N-triméthyl 3-[(1-oxo 2-propènyl) amino] propanammonium (APTAC),
a2) de 0,1 % molaire à 5 % molaire d'unités monomériques issues d'au moins un monomère de formule (VI) : 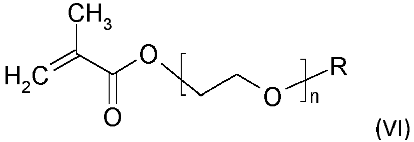 dans laquelle R représente un radical alkyle linéaire ou ramifié comportant de huit à vingt atomes de carbone et n représente un nombre entier supérieur ou égal à zéro et inférieur ou égal à vingt ;
a3) de 35 % molaire à 94,9 % molaire d'unités monomériques issues d'au moins un monomère neutre choisi parmi les éléments du groupe constitué par l'acrylamide, le N,N-diméthyl acrylamide, le méthacrylamide, le N-isopropyl acrylamide, le 2-hydroxyétyl acrylate ou le 2-hydroxyéthyl méthacrylate ;
a4) d'une proportion supérieure à 0% molaire et inférieure ou égale à 1% molaire d'unités monomériques issues d'au moins un monomère de réticulation (AR) diéthylénique ou polyéthylénique ;
la somme des dites proportions molaires en unités monomériques selon a1), a2), a3) et a4) étant égale à 100% molaire.

4. Composition cosmétique (C1) telle que définie à la revendication 3, **caractérisée en ce que** ledit monomère neutre est le 2-hydroxyéthyl acrylate.

5. Composition cosmétique (C1) telle que définie à la revendication 3, **caractérisée en ce que** ledit monomère de formule (VI) est le méthacrylate de lauryle tétraéthoxylé.

6. Composition cosmétique (C1) telle que définie à l'une ou quelconque des revendications 1 à 5, **caractérisée en ce que** ledit polyélectrolyte cationique réticulé (PC) est un terpolymère de du chlorure de N,N,N-triméthyl 3-[(1-oxo 2-propènyl) amino] propanammonium (APTAC), du 2-hydroxyéthyl acrylate et du méthacrylate de lauryle tétraéthoxylé, réticulé au triméthylol propanetriacrylate ou au diméthacrylate d'éthylèneglycol ou au diacrylate d'éthylèneglycol.

7. Composition (C₁) telle que définie à l'une ou quelconque des revendications 1 à 6, **caractérisée en ce que** ledit polyélectrolyte cationique réticulé (PC) comporte pour 100% molaire :
- De 45% molaire à 60% molaire d'unités monomériques issues du chlorure de N,N,N-triméthyl 3-[(1-oxo 2-propènyl) amino] propanammonium
- De 0,5% molaire à 3% molaire d'unités monomériques issues du méthacrylate de lauryle tétraéthoxylé, et
- De 37% molaire à 54,5% molaire d'unités monomériques issues du 2-hydroxyéthyl acrylate.

8. Composition cosmétique (C1) telle que définie à l'une ou quelconque des revendications 1 à 7, **caractérisée en ce que** ledit système émulsionnant (S) consiste en une composition (C2) d'alkylpolyglycosides représentée par la formule (VII) :
R₁-O-(G)ₓ-H (VII)
dans laquelle x représente un nombre décimal compris entre 1,05 et 2,5, G représente le reste du xylose, et R₁ représente le radical 2-octyl dodécyle, ladite composition (C₁) consistant en un mélange de composés représentés par les formules (VII₁), (VII₂), (VII₃), (VII₄) et (VII₅) :
R₁-O-(G)₁-H (VII₁)
R₁-O-(G)₂-H (VII₂)
R₁-O-(G)₃-H (VII₃)
R₁-O-(G)₄-H (VII₄)
R₁-O-(G)₅-H (VII₅)
dans les proportions molaires respectives a₁, a₂, a₃, a₄ et a₅, telles que :
- La somme a₁+ a₂ + a₃ + a₄ + a₅ est égale à 1 et que
- La somme a₁ + 2a₂ + 3a₃ + 4a₄ + 5as est égale à x.

9. Composition cosmétique (C1) telle que définie à l'une ou quelconque des revendications 1 à 7, **caractérisée en ce que** ledit système émulsionnant (S) consiste en une composition (C3) comprenant pour 100% de sa masse :
- De 10 % à 50 % massique d'au moins une composition d'alkylpolyglycosides (C2) représentée par la formule (VII) :
R₁-O-(G)ₓ-H (VII)
dans laquelle x représente un nombre décimal compris entre 1,05 et 2,5, G représente le reste du xylose et R₁ représente le radical 2-octyl dodécyle, ladite composition consistant en un mélange de composés représentés par les formules (VII₁), (VII₂), (VII₃), (VII₄) et (VII₅) :
R₁-O-(G)₁-H (VII₁)
R₁-O-(G)₂-H (VII₂)
R₁-O-(G)₃-H (VII₃)
R₁-O-(G)₄-H (VII₄)
R₁-O-(G)₅-H (VII₅)
dans les proportions molaires respectives a₁, a₂, a₃, a₄ et as, telles que :
- La somme a₁+ a₂ + a₃ + a₄ + as est égale à 1 et que
- La somme a₁ + 2a₂ + 3a₃ + 4a₄ + 5as est égale à x ; et
- De 90% à 50% massique d'au moins un alcool gras de formule (VIII) :
R'₁-OH (VIII),
dans laquelle R'₁ représente le radical 2-octyl dodécyle.

10. Composition cosmétique (C1) telle que définie à l'une ou quelconque des revendications 1 à 7, **caractérisée en ce que** ledit système émulsionnant (S) consiste en une composition (C4) comprenant pour 100% de sa masse :
De 15 % à 25 % massique d'au moins une composition (C2) représentée par la formule (VII) :
R1-O-(G)ₓ-H (VII)
dans laquelle x représente un nombre décimal compris entre 1,05 et 2,5, G représente le reste du xylose et R1 représente le radical 2-octyl dodécyle, ladite composition (C₁) consistant en un mélange de composés représentés par les formules (VII₁), (VII₂), (VII₃), (VII₄) et (VII₅) :
R₁-O-(G)₁-H (VII₁)
R₁-O-(G)₂-H (VII₂)
R₁-O-(G)₃-H (VII₃)
R₁-O-(G)₄-H (VII₄)
R₁-O-(G)₅-H (VII₅)
dans les proportions molaires respectives a₁, a₂, a₃, a₄ et a₅, telles que :
- La somme a₁+ a₂ + a₃ + a₄ + a₅ est égale à 1 et que
- La somme a₁ + 2a₂ + 3a₃ + 4a₄ + 5as est égale à x ;
De 55 % à 65 % massique d'au moins un alcool gras de formule (VIII) :
R'₁-OH (VIII),
dans laquelle R'₁ représente le radical 2-octyl dodécyle ;
De 10 % à 30 % massique d'au moins un polyhydroxystéarates de polyglycols représenté par la formule (IX) : 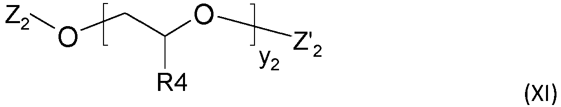 dans laquelle y₂ représente un nombre entier supérieur ou égal à 2 et inférieur ou égal à 50, R₄ représente l'atome d'hydrogène, le radical méthyle ou le radical éthyle, Z₂ représente un radical de formule (XII) : 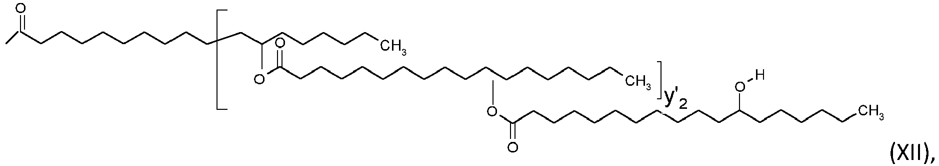 dans laquelle y'₂ représente un nombre entier supérieur ou égal à 0 et inférieur ou égal à 10, plus particulièrement supérieur ou égal à 1 et inférieur ou égal à 10, Z'₂ représente un radical de formule (XII) telle que définie ci-dessus, avec Z₂' identique ou différent de Z₂, ou l'atome d'hydrogène.

11. Polyélectrolyte cationique réticulé (PC1) comprenant pour 100% molaire :
b1) de 40% à 60% molaire d'unités monomériques cationiques issues d'un élément du groupe constitué par le chlorure de 2,N,N,N-tétraméthyl 2-[(1-oxo 2-propènyl) amino] propanammonium (AMPTAC), le chlorure de 2,N,N-triméthyl 2-[(1-oxo 2-propènyl) amino] propanammonium, le chlorure de N,N,N-triméthyl 3-[(1-oxo 2-propènyl) amino] propanammonium (APTAC),
b2) de 0,1% à 3% molaire d'unités monomériques issues d'au moins un monomère de formule (VI) : 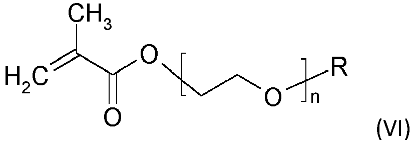 dans laquelle R représente un radical alkyle linéaire ou ramifié comportant de huit à vingt atomes de carbone et n représente un nombre entier supérieur ou égal à zéro et inférieur ou égal à vingt ;
b3) de 37% à 59,9% molaire d'unités monomériques issues d'au moins un monomère neutre choisi parmi les éléments du groupe constitué par l'acrylamide, le N,N-diméthyl acrylamide ; le méthacrylamide ou le N-isopropyl acrylamide le 2-hydroxyétyl acrylate ou le 2-hydroxyéthyl méthacrylate ;
b4) d'une proportion supérieure à 0% molaire et inférieure ou égale à 1% molaire d'unités monomériques issues d'au moins un monomère de réticulation (AR) diéthylénique ou polyéthylénique ;
la somme des dites proportions molaires en unités monomériques selon b1), b2), b3) et b4) étant égale à 100% molaire.

12. Polyélectrolyte cationique réticulé (PC1) tel que définie à la revendication 11, **caractérisé en ce qu'**il comporte pour 100% molaire :
- De 45% molaire à 60% molaire d'unités monomériques issues du chlorure de N,N,N-triméthyl 3-[(1-oxo 2-propènyl) amino] propanammonium
- De 0,5% molaire à 3% molaire d'unités monomériques issues du méthacrylate de lauryle tétraéthoxylé, et
- De 37% molaire à 54,5% molaire d'unités monomériques issues du 2-hydroxyéthyl acrylate.

13. Utilisation d'une composition cosmétique (C1) telle que définie à l'une ou quelconque des revendications 1 à 10 pour améliorer le peignage de mèches de cheveux

## Patentansprüche

1. Kosmetische Zusammensetzung (C1) in Form einer Emulsion vom Wasser-in-Öl-Typ, umfassend pro 100 % ihrer Masse:
- 60 bis 95 Massen-% einer gelierten wässrigen Phase (A1), umfassend mindestens einen vernetzten kationischen Polyelektrolyt, der einen Anteil größer oder gleich 5 Mol-% von Monomereinheiten, die sich von einem Element der Gruppe bestehend aus 2,N,N,N-Tetramethyl-2-[(1-oxo-2-propenyl)amino]propanammoniumchlorid (AMPTAC), 2,N,N-Trimethyl-2-[(1-oxo-2-propenyl)amino]propanammonium-chlorid und N,N,N-Trimethyl-3-[(1-oxo-2-propenyl)amino]-propanammoniumchlorid (APTAC) ableiten, umfasst,
- 5 bis 40 Massen-% einer Fettphase (A2), umfassend mindestens ein Öl und ein Emulgiersystem, das eine Kombination von mindestens einem emulgierenden Tensid (S), das aus den Elementen der Gruppe bestehend aus Zusammensetzungen von Alkylpolyglycosiden und Zusammensetzungen von Alkylpolyglycosiden und Fettalkoholen, Polyglycerinestern, alkoxylierten Polyglycerinestern, Polyglykolpolyhydroxystearaten, Polyglycerinpoly-hydroxystearaten und alkoxylierten Polyglycerinpoly-hydroxystearaten ausgewählt ist, umfasst.

2. Zusammensetzung (C1) nach Anspruch 1, **dadurch gekennzeichnet, dass** die gelierte wässrige Phase (A1) pro 100 % ihrer Masse 0,1 bis 7 Massen-% eines vernetzten kationischen Polyelektrolyts (PC) und 93 bis 99,9 Massen-% Wasser umfasst.

3. Zusammensetzung (C1) nach Anspruch 1 oder 2, **dadurch gekennzeichnet, dass** der vernetzte kationische Polyelektrolyt (PC) pro 100 Mol-% Folgendes umfasst:
a1) 5 Mol-% bis 60 Mol-% von Monomereinheiten, die sich von einem Element der Gruppe bestehend aus 2,N,N,N-Tetramethyl-2-[(1-oxo-2-propenyl)amino]propanammonium-chlorid (AMPTAC), 2,N,N-Trimethyl-2-[(1-oxo-2-propenyl)-amino]propanammoniumchlorid und N,N,N-Trimethyl-3-[(1-oxo-2-propenyl)amino]propanammoniumchlorid (APTAC) ableiten,
a2) 0,1 Mol-% bis 5 Mol-% von Monomereinheiten, die sich von mindestens einem Monomer der Formel (VI) ableiten: 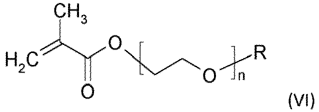 wobei R für einen linearen oder verzweigten Alkylrest mit acht bis zwanzig Kohlenstoffatomen steht und n für eine ganze Zahl größer oder gleich null und kleiner oder gleich zwanzig steht;
a3) 35 Mol-% bis 94,9 Mol-% von Monomereinheiten, die sich von mindestens einem neutralen Monomer, das aus den Elementen der Gruppe bestehend aus Acrylamid, N,N-Dimethylacrylamid, Methacrylamid, N-Isopropylacrylamid, 2-Hydroxyethylacrylat und 2-Hydroxyethylmethacrylat ausgewählt ist, ableiten;
a4) einen Anteil größer als 0 Mol-% und kleiner oder gleich 1 Mol-% von Monomereinheiten, die sich von mindestens einem diethylenischen oder polyethylenischen Vernetzungsmonomer (AR) ableiten;
wobei die Summe der molaren Anteile an Monomereinheiten gemäß a1), a2), a3) und a4) gleich 100 Mol-% ist.

4. Kosmetische Zusammensetzung (C1) nach Anspruch 3, **dadurch gekennzeichnet, dass** es sich bei dem neutralen Monomer um 2-Hydroxyethylacrylat handelt.

5. Kosmetische Zusammensetzung (C1) nach Anspruch 3, **dadurch gekennzeichnet, dass** es sich bei dem Monomer der Formel (VI) um tetraethoxyliertes Laurylmethacrylat handelt.

6. Kosmetische Zusammensetzung (C1) nach einem der Ansprüche 1 bis 5, **dadurch gekennzeichnet, dass** es sich bei dem vernetzten kationischen Polyelektrolyt (PC) um ein mit Trimethylolpropantriacrylat oder mit Ethylenglykoldimethacrylat oder mit Ethylenglykoldiacrylat vernetztes Terpolymer von N,N,N-Trimethyl-3-[(1-oxo-2-propenyl)amino]propanammoniumchlorid (APTAC), 2-Hydroxyethylacrylat und tetraethoxyliertem Laurylmethacrylat handelt.

7. Zusammensetzung (C1) nach einem der Ansprüche 1 bis 6, **dadurch gekennzeichnet, dass** der vernetzte kationische Polyelektrolyt (PC) pro 100 Mol-% Folgendes umfasst:
- 45 Mol-% bis 60 Mol-% von Monomereinheiten, die sich von N,N,N-Trimethyl-3-[(1-oxo-2-propenyl)amino]propan-ammoniumchlorid ableiten,
- 0,5 Mol-% bis 3 Mol-% von Monomereinheiten, die sich von tetraethoxyliertem Laurylmethacrylat ableiten, und
- 37 Mol-% bis 54,5 Mol-% von Monomereinheiten, die sich von 2-Hydroxyethylacrylat ableiten.

8. Zusammensetzung (C1) nach einem der Ansprüche 1 bis 7, **dadurch gekennzeichnet, dass** das Emulgiersystem (S) aus einer Zusammensetzung (C2) von Alkylpolyglycosiden besteht, die durch die Formel (VII) wiedergegeben wird:
R₁-O-(G)ₓ-H (VII)
wobei x für eine Dezimalzahl zwischen 1,05 und 2,5 steht, G für einen Xyloserest steht und R₁ für einen 2-Octyl-dodecylrest steht, wobei die Zusammensetzung (C1) aus einem Gemisch von Verbindungen besteht, die durch die Formeln (VII₁), (VII₂), (VII₃), (VII₄) und (VII₅) wiedergegeben werden:
R₁-O-(G)₁-H (VII₁)
R₁-O-(G)₂-H (VII₂)
R₁-O-(G)₃-H (VII₃)
R₁-O-(G)₄-H (VII₄)
R₂-O-(G)₅-H (VII₅)
in solchen jeweiligen molaren Anteilen a₁, a₂, a₃, a₄ und a₅, dass:
- die Summe a₁ + a₂ + a₃ + a₄ + a₅ gleich 1 ist und dass
- die Summe a₁ + 2a₂ + 3a₃ + 4a₄ + 5a₅ gleich x ist.

9. Kosmetische Zusammensetzung (C1) nach einem der Ansprüche 1 bis 7, **dadurch gekennzeichnet, dass** das Emulgiersystem (S) aus einer Zusammensetzung (C3) besteht, die pro 100 % ihrer Masse Folgendes umfasst:
- 10 bis 50 Massen-% mindestens einer Zusammensetzung von Alkylpolyglycosiden (C2), die durch die Formel (VII) wiedergegeben wird:
R₁-O-(G)ₓ-H (VII)
wobei x für eine Dezimalzahl zwischen 1,05 und 2,5 steht, G für einen Xyloserest steht und R₁ für einen 2-Octyl-dodecylrest steht, wobei die Zusammensetzung aus einem Gemisch von Verbindungen besteht, die durch die Formeln (VII₁), (VII₂), (VII₃), (VII₄) und (VII₅) wiedergegeben werden:
R₁-O-(G)₁-H (VII₁)
R₁-O-(G)₂-H (VII₂)
R₁-O-(G)₃-H (VII₃)
R₁-O-(G)₄-H (VII₄)
R₂-O-(G)₅-H (VII₅)
in solchen jeweiligen molaren Anteilen a₁, a₂, a₃, a₄ und a₅, dass:
- die Summe a₁ + a₂ + a₃ + a₄ + a₅ gleich 1 ist und dass
- die Summe a₁ + 2a₂ + 3a₃ + 4a₄ + 5a₅ gleich x ist; und
- 90 bis 50 Massen-% mindestens eines Fettalkohols der Formel (VIII):
R'₁-OH (VIII),
wobei R'₁ für einen 2-Octyldodecylrest steht.

10. Kosmetische Zusammensetzung (C1) nach einem der Ansprüche 1 bis 7, **dadurch gekennzeichnet, dass** das Emulgiersystem (S) aus einer Zusammensetzung (C4) besteht, die pro 100 % ihrer Masse Folgendes umfasst: 15 bis 25 Massen-% mindestens einer Zusammensetzung (C2), die durch die Formel (VII) wiedergegeben wird: R₁-O-(G)ₓ-H (VII) wobei x für eine Dezimalzahl zwischen 1,05 und 2,5 steht, G für einen Xyloserest steht und R₁ für einen 2-Octyl-dodecylrest steht, wobei die Zusammensetzung (C1) aus einem Gemisch von Verbindungen besteht, die durch die Formeln (VII₁), (VII₂), (VII₃), (VII₄) und (VII₅) wiedergegeben werden:
R₁-O-(G)₁-H (VII₁)
R₁-O-(G)₂-H (VII₂)
R₁-O-(G)₃-H (VII₃)
R₁-O-(G)₄-H (VII₄)
R₂-O-(G)₅-H (VII₅)
in solchen jeweiligen molaren Anteilen a₁, a₂, a₃, a₄ und a₅, dass:
- die Summe a₁ + a₂ + a₃ + a₄ + a₅ gleich 1 ist und dass
- die Summe a₁ + 2a₂ + 3a₃ + 4a₄ + 5a₅ gleich x ist; und 55 bis 65 Massen-% mindestens eines Fettalkohols der Formel (VIII):
R'₁-OH (VIII),
wobei R'₁ für einen 2-Octyldodecylrest steht; 10 bis 30 Massen-% mindestens eines Polyglykol-polyhydroxystearats, das durch die Formel (IX) wiedergegeben wird: 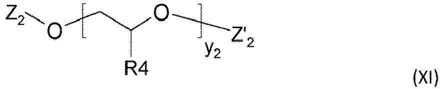 wobei y₂ für eine ganze Zahl größer oder gleich 2 und kleiner oder gleich 50 steht, R₄ für ein Wasserstoffatom, den Methylrest oder den Ethylrest steht und Z₂ für einen Rest der Formel (XII) steht: 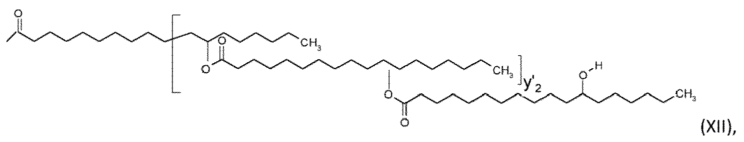 wobei y'₂ für eine ganze Zahl größer oder gleich 0 und kleiner oder gleich 10, spezieller größer oder gleich 1 und kleiner oder gleich 10, steht und Z'₂ für einen Rest der Formel (XII) gemäß obiger Definition steht, wobei Z'₂ mit Z₂ identisch oder davon verschieden sein kann, oder ein Wasserstoffatom ist.

11. Vernetzter kationischer Polyelektrolyt (PC1), umfassend pro 100 Mol-%: b1) 40 Mol-% bis 60 Mol-% von kationischen Monomereinheiten, die sich von einem Element der Gruppe bestehend aus 2,N,N,N-Tetramethyl-2-[(1-oxo-2-propenyl)-amino]propanammoniumchlorid (AMPTAC), 2,N,N-Trimethyl-2-[(1-oxo-2-propenyl)amino]propanammoniumchlorid und N,N,N-Trimethyl-3-[(1-oxo-2-propenyl)amino]propan-ammoniumchlorid (APTAC) ableiten, b2) 0,1 Mol-% bis 3 Mol-% von Monomereinheiten, die sich von mindestens einem Monomer der Formel (VI) ableiten: 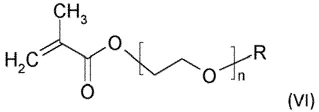
wobei R für einen linearen oder verzweigten Alkylrest mit acht bis zwanzig Kohlenstoffatomen steht und n für eine ganze Zahl größer oder gleich null und kleiner oder gleich zwanzig steht;
b3) 37 Mol-% bis 59,9 Mol-% von Monomereinheiten, die sich von mindestens einem neutralen Monomer, das aus den Elementen der Gruppe bestehend aus Acrylamid, N,N-Dimethylacrylamid, Methacrylamid, N-Isopropylacrylamid, 2-Hydroxyethylacrylat und 2-Hydroxyethylmethacrylat ausgewählt ist, ableiten;
b4) einen Anteil größer als 0 Mol-% und kleiner oder gleich 1 Mol-% von Monomereinheiten, die sich von mindestens einem diethylenischen oder polyethylenischen Vernetzungsmonomer (AR) ableiten;
wobei die Summe der molaren Anteile an Monomereinheiten gemäß b1), b2), b3) und b4) gleich 100 Mol-% ist.

12. Vernetzter kationischer Polyelektrolyt (PC1) nach Anspruch 11, **dadurch gekennzeichnet, dass** er pro 100 Mol-% Folgendes umfasst:
- 45 Mol-% bis 60 Mol-% von Monomereinheiten, die sich von N,N,N-Trimethyl-3-[(1-oxo-2-propenyl)amino]propan-ammoniumchlorid ableiten,
- 0,5 Mol-% bis 3 Mol-% von Monomereinheiten, die sich von tetraethoxyliertem Laurylmethacrylat ableiten, und
- 37 Mol-% bis 54,5 Mol-% von Monomereinheiten, die sich von 2-Hydroxyethylacrylat ableiten.

13. Verwendung einer kosmetischen Zusammensetzung (C1) nach einem der Ansprüche 1 bis 10 zur Verbesserung des Kämmens von Haarsträhnen.

## Claims

1. Cosmetic composition (C1) in the form of an emulsion of water-in-oil type comprising, per 100% of its mass:
- from 60% to 95% by mass of a gelled aqueous phase (A1) comprising at least one crosslinked cationic polyelectrolyte which comprises a proportion of greater than or equal to 5 mol% of monomer units derived from an element from the group consisting of 2,N,N,N-tetramethyl-2-[(1-oxo-2-propenyl)amino]propanammonium chloride (AMPTAC), 2,N,N-trimethyl-2-[(1-oxo-2-propenyl)amino]propanammonium chloride and N,N,N-trimethyl-3-[(1-oxo-2-propenyl)amino]propanammonium chloride (APTAC),
- from 5% to 40% by mass of a fatty phase (A2) comprising at least one oil and an emulsifying system comprising a combination of at least one emulsifying surfactant (S) selected from the elements of the group consisting of compositions of alkylpolyglycosides, and compositions of alkylpolyglycosides and of fatty alcohols, polyglycerol esters, alkoxylated polyglycerol esters, polyglycol polyhydroxystearates, polyglycerol polyhydroxystearates, and alkoxylated polyglycerol polyhydroxystearates.

2. Composition (C1) as defined in Claim 1, **characterized in that** the gelled aqueous phase (A1) comprises, per 100% of its mass, from 0.1% to 7% by mass of a crosslinked cationic polyelectrolyte (CP) and from 93% to 99.9% by mass of water.

3. Composition (C1) as defined in either of Claims 1 and 2, **characterized in that** the crosslinked cationic polyelectrolyte (CP) comprises, per 100 mol%:
a1) from 5 mol% to 60 mol% of monomer units derived from an element from the group consisting of 2,N,N,N-tetramethyl-2-[(1-oxo-2-propenyl)amino]propanammonium chloride (AMPTAC), 2,N,N-trimethyl-2-[(1-oxo-2-propenyl)amino]propanammonium chloride and N,N,N-trimethyl-3-[(1-oxo-2-propenyl)amino]propanammonium chloride (APTAC),
a2) from 0.1 mol% to 5 mol% of monomer units derived from at least one monomer of formula (VI): 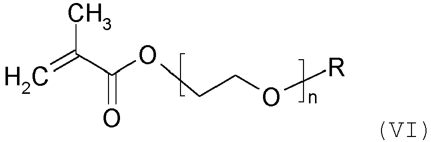 in which R represents a linear or branched alkyl radical including from 8 to 20 carbon atoms and n represents an integer greater than or equal to 0 and less than or equal to 20;
a3) from 35 mol% to 94.9 mol% of monomer units derived from at least one neutral monomer chosen from the elements of the group consisting of acrylamide, N,N-dimethylacrylamide, methacrylamide, N-isopropylacrylamide, 2-hydroxyethyl acrylate and 2-hydroxyethyl methacrylate;
a4) a proportion of greater than 0 mol% and less than or equal to 1 mol% of monomer units derived from at least one diethylenic or polyethylenic crosslinking monomer (AR) ;
the sum of said molar proportions of monomer units according to a1), a2), a3) and a4) being equal to 100 mol%.

4. Cosmetic composition (C1) as defined in Claim 3, **characterized in that** said neutral monomer is 2-hydroxyethyl acrylate.

5. Cosmetic composition (C1) as defined in Claim 3, **characterized in that** said monomer of formula (VI) is tetraethoxylated lauryl methacrylate.

6. Cosmetic composition (C1) as defined in any one of Claims 1 to 5, **characterized in that** said crosslinked cationic polyelectrolyte (CP) is a terpolymer of N,N,N-trimethyl-3-[(1-oxo-2-propenyl)amino]propanammonium chloride (APTAC), of 2-hydroxyethyl acrylate and of tetraethoxylated lauryl methacrylate crosslinked with trimethylolpropane triacrylate or with ethylene glycol dimethacrylate or with ethylene glycol diacrylate.

7. Composition (C₁) as defined in any one of Claims 1 to 6, **characterized in that** said crosslinked cationic polyelectrolyte (CP) includes, per 100 mol%:
- from 45 mol% to 60 mol% of monomer units derived from N,N,N-trimethyl-3-[(1-oxo-2-propenyl)amino]propanammonium chloride
- from 0.5 mol% to 3 mol% of monomer units derived from tetraethoxylated lauryl methacrylate, and
- from 37 mol% to 54.5 mol% of monomer units derived from 2-hydroxyethyl acrylate.

8. Cosmetic composition (C1) as defined in any one of Claims 1 to 7, **characterized in that** said emulsifying system (S) consists of an alkylpolyglycoside composition (C2) represented by formula (VII):
R₁-O-(G)ₓ-H (VII)
in which x represents a decimal number between 1.05 and 2.5, G represents a xylose residue, and R₁ represents a 2-octyldodecyl radical, said composition (C₁) consisting of a mixture of compounds represented by formulae (VII₁), (VII₂), (VII₃), (VII₄) and (VII₅):
R₁-O-(G)₁-H (VII₁)
R₁-O-(G)₂-H (VII₂)
R₁-O-(G)₃-H (VII₃)
R₁-O-(G)₄-H (VII₄)
R₁-O-(G)₅-H (VII₅)
in the respective molar proportions a₁, a₂, a₃, a₄ and a₅, such that:
- the sum a₁ + a₂ + a₃ + a₄ + a₅ is equal to 1, and that
- the sum a₁ + 2a₂ + 3a₃ + 4a₄ + 5a₅ is equal to x.

9. Cosmetic composition (C1) as defined in any one of Claims 1 to 7, **characterized in that** said emulsifying system (S) consists of a composition (C3) comprising, per 100% of its mass:
- from 10% to 50% by mass of at least one alkylpolyglycoside composition (C2) represented by formula (VII):
R₁-O-(G)ₓ-H (VII)
in which x represents a decimal number between 1.05 and 2.5, G represents a xylose residue, and R₁ represents a 2-octyldodecyl radical, said composition consisting of a mixture of compounds represented by formulae (VII₁), (VII₂), (VII₃), (VII₄) and (VII₅):
R₁-O-(G)₁-H (VII₁)
R₁-O-(G)₂-H (VII₂)
R₁-O-(G)₃-H (VII₃)
R₁-O-(G)₄-H (VII₄)
R₂-O-(G)₅-H (VII₅)
in the respective molar proportions a₁, a₂, a₃, a₄ and a₅, such that:
- the sum a₁ + a₂ + a₃ + a₄ + a₅ is equal to 1, and that
- the sum a₁ + 2a₂ + 3a₃ + 4a₄ + 5a₅ is equal to x; and
- from 90% to 50% by mass of at least one fatty alcohol of formula (VIII): R'₁-OH (VIII), in which R'₁ represents a 2-octyldodecyl radical.

10. Cosmetic composition (C1) as defined in any one of Claims 1 to 7, **characterized in that** said emulsifying system (S) consists of a composition (C4) comprising, per 100% of its mass:
from 15% to 25% by mass of at least one composition (C2) represented by formula (VII):
R1-O-(G)ₓ-H (VII)
in which x represents a decimal number between 1.05 and 2.5, G represents a xylose residue, and R1 represents a 2-octyldodecyl radical, said composition (C₁) consisting of a mixture of compounds represented by formulae (VII₁), (VII₂), (VII₃), (VII₄) and (VII₅):
R₁-O-(G)₁-H (VII₁)
R₁-O-(G)₂-H (VII₂)
R₁-O-(G)₃-H (VII₃)
R₁-O-(G)₄-H (VII₄)
R₂-O-(G)₅-H (VII₅)
in the respective molar proportions a₁, a₂, a₃, a₄ and a₅, such that:
- the sum a₁ + a₂ + a₃ + a₄ + a₅ is equal to 1, and that
- the sum a₁ + 2a₂ + 3a₃ + 4a₄ + 5a₅ is equal to x; from 55% to 65% by mass of at least one fatty alcohol of formula (VIII):
R'₁-OH (VIII),
in which R'₁ represents a 2-octyldodecyl radical; from 10% to 30% by mass of at least one polyglycol polyhydroxystearate represented by formula (IX): 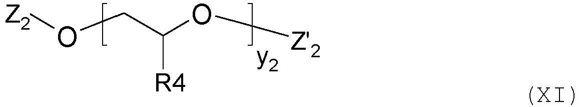 in which y₂ represents an integer greater than or equal to 2 and less than or equal to 50, R₄ represents a hydrogen atom, a methyl radical or an ethyl radical, and Z₂ represents a radical of formula (XII): 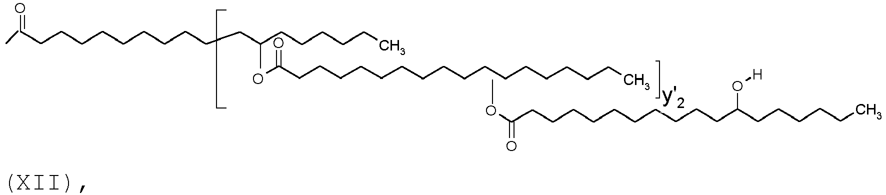 in which y'₂ represents an integer greater than or equal to 0 and less than or equal to 10, more particularly greater than or equal to 1 and less than or equal to 10, and Z'₂ represents a radical of formula (XII) as defined above, where Z₂' may be identical to or different from Z₂, or a hydrogen atom.

11. Crosslinked cationic polyelectrolyte (CP1) comprising, per 100 mol%:
b1) from 40 mol% to 60 mol% of cationic monomer units derived from an element from the group consisting of 2,N,N,N-tetramethyl-2-[(1-oxo-2-propenyl)amino]propanammonium chloride (AMPTAC), 2,N,N-trimethyl-2-[(1-oxo-2-propenyl)amino]propanammonium chloride and N,N,N-trimethyl-3-[(1-oxo-2-propenyl)amino]propanammonium chloride (APTAC),
b2) from 0.1 mol% to 3 mol% of monomer units derived from at least one monomer of formula (VI): 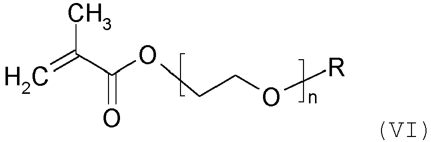 in which R represents a linear or branched alkyl radical including from 8 to 20 carbon atoms and n represents an integer greater than or equal to 0 and less than or equal to 20;
b3) from 37 mol% to 59.9 mol% of monomer units derived from at least one neutral monomer chosen from the elements of the group consisting of acrylamide, N,N-dimethylacrylamide, methacrylamide or N-isopropylacrylamide, 2-hydroxyethyl acrylate or 2-hydroxyethyl methacrylate;
b4) a proportion of greater than 0 mol% and less than or equal to 1 mol% of monomer units derived from at least one diethylenic or polyethylenic crosslinking monomer (AR) ;
the sum of said molar proportions of monomer units according to b1), b2), b3) and b4) being equal to 100 mol%.

12. Crosslinked cationic polyelectrolyte (CP1) as defined in Claim 11, **characterized in that** it includes, per 100 mol%:
- from 45 mol% to 60 mol% of monomer units derived from N,N,N-trimethyl-3-[(1-oxo-2-propenyl)amino]propanammonium chloride
- from 0.5 mol% to 3 mol% of monomer units derived from tetraethoxylated lauryl methacrylate, and
- from 37 mol% to 54.5 mol% of monomer units derived from 2-hydroxyethyl acrylate.

13. Use of a cosmetic composition (C1) as defined in any one of Claims 1 to 10, for improving the combing of locks of hair.
